# EUROPEAN PATENT APPLICATION

(11) **EP 2 755 029 A1**
(43) Date of publication of application: **16.07.2014**
(21) Application number: 12820586.1
(22) Date of filing: 01.08.2012
(51) Int. Cl.: G01N 33/543, G01N 33/53, C07K 14/37, C12N 15/09

(54) **METHOD FOR INHIBITING NON-SPECIFIC BINDING IN STEP OF DETECTING SUBSTANCE IN BIOLOGICAL SAMPLE, AND AGENT FOR USE IN THE METHOD**

(30) Priority: 01.08.2011 JP 2011168422
(71) Applicant: Japan Tobacco, Inc., Tokyo 105-8422 (JP)
(72) Inventor: TAKAKURA, Yoshimitsu, Iwata-shi Shizuoka 438-0802 (JP); OKA, Naomi, Iwata-shi Shizuoka 438-0802 (JP)
(74) Representative: Vossius & Partner
(86) International application number: PCT/JP2012/069607
(87) International publication number: WO 2013/018836

(57) **Abstract**

The present invention provides a method of inhibiting non-specific binding in a step of detecting a substance in a biological sample. The invention provides a method of inhibiting non-specific binding in a step of detecting a substance in a biological sample, the method comprising:
bringing a biotin-binding protein monomer not substantially having biotin-binding ability into contact with the biological sample, wherein
in the step of detecting, a substance capable of specifically binding to the substance to be detected is immobilized to a carrier through binding between biotin and the biotin-binding protein before or after binding of the substance to be detected to the substance capable of specifically binding to the substance to be detected.

## Description

### TECHNICAL FIELD

The present application claims priority to Japanese Patent Application No. 2011-168422 filed Aug. 1, 2011, the entire contents of which are incorporated herein by reference.

The present invention relates to a method of inhibiting non-specific binding in a step of detecting a substance in a biological sample and relates to an agent for use in the method.

### BACKGROUND ART

### Biotin-binding protein

Avidin is a basic glycoprotein derived from albumen and strongly binds to biotin (vitamin H). Avidin has an isoelectric point of higher than 10 and may cause a problem of non-specific binding to biological molecules such as DNAs and proteins because of its high basicity or the presence of a sugar chain. Streptavidin is an avidin-like protein derived from Streptomyces avidinii and does not have a sugar chain. Streptavidin has an approximately neutral isoelectric point and also strongly binds to biotin.

Avidin and streptavidin each form a tetramer having a molecular weight of about 60 kDa. The tetramer is composed of two dimers weakly bound to each other, and the dimers are each composed of two monomers strongly bound to each other. Each monomer of avidin and streptavidin binds to one biotin molecule. The affinities between avidin and biotin and between streptavidin and biotin are very high (Kd = 10⁻¹⁵ to 10⁻¹⁴ M) and are the highest as interaction between two biomolecules.

Tamavidin 1 and tamavidin 2 are novel avidin-like biotin-binding proteins that have been discovered by the present inventors in an edible mushroom (Pleurotus comucopiae) (WO02/072817). Both of them can be expressed in E. coli, and large amounts thereof can be found in soluble protein fractions. In particular, tamavidin 2 can be easily prepared by purification through an iminobiotin column. Tamavidin 1 and tamavidin 2 each form a tetramer as in avidin and streptavidin and significantly strongly bind to biotin. Tamavidin 2 is a superior biotin-binding protein having a heat resistance higher than that of avidin or streptavidin and non-specific binding less than that of avidin.

### Immobilization, to a carrier, of a substance specifically binding to substance to be detected in a biological sample using biotin-binding protein

In order to detect a substance to be detected in a biological sample, a substance specifically binds to the substance to be detected has been widely used. For example, in an immunoassay, an antigen for detecting an antibody as the substance to be detected or an antibody for detecting an antigen as the substance to be detected is immobilized to a carrier, such as a microplate, microbeads, or a sensor chip, before or after the reaction with the substance to be detected, and the presence or the degree of an antigen-antibody reaction is detected or measured. In such a case, strong interaction between a biotin-binding protein and biotin is often used for the immobilization of a protein.

In immobilization using a biotin-binding protein of a substance (e.g., a protein) specifically binding to a substance to be detected to a carrier, the biotin-binding protein is immobilized to the carrier, such as a microplate or microbeads, through covalent binding or hydrophobic binding, and a biotinylated substance capable of specifically binding to the substance to be detected is then bound to the biotin-binding protein. The biotinylated substance capable of specifically binding to a substance to be detected may be immobilized to a biotin-binding protein-immobilized carrier and then subjected to a reaction with a biological sample, or may be subjected to a reaction with a biological sample and then immobilized to a biotin-binding protein-immobilized carrier.

A technology of immobilizing biotin, a biotin-binding protein, biotin, and a substance capable of specifically binding to a substance to be detected in this order to a base plate is reported in which the biotin-binding protein is bound to the biotin-bound base plate by a biotin-binding protein-biotin binding and then a biotinylated substance capable of specifically binding to the substance to be detected is bound at another biotin pocket of the biotin-binding protein (JP H04-236353 A). Furthermore, a fusion protein composed of a substance capable of specifically binding to a substance to be detected and a biotin-binding protein may be immobilized to a biotin-immobilized carrier, before or after the reaction with a biological sample.

### Step of detecting a substance in a biological sample

The carrier to which a substance capable of specifically binding to a substance to be detected is bound through binding between biotin and a biotin-binding protein can be used in a step of detecting the substance in a biological sample. Such an experimental system, however, can involve not only specific binding reactions, such as a reaction between a desired substance to be detected and a substance specifically binding to the substance to be detected or a reaction between a biotin-binding protein and biotin, but also undesired non-specific binding reactions, e.g., reactions between substances other than the substance to be detected in a biological sample and the substances constituting the solid phase. Such non-specific interactions cause an increase in background signal and an increase in signal scattering, resulting in a decrease in detection sensitivity.

In order to solve this problem, the following methods have been proposed for example: a method of adding, to an agent for detection, an extract of a bacterium component used in production of a recombinant protein capable of specifically binding to a substance to be detected (JP S59-99257 A); a method of adding, to a sample, a culture component of host cells containing a vector of the same species as that used in production of a recombinant protein capable of specifically binding to a substance to be detected and not containing a gene encoding the protein (JP H08-43392 A); and a method of heat-treating an extract from cells of the same species as that producing a recombinant protein capable of specifically binding to a substance to be detected and not containing the protein, and then adding its water-soluble fraction to a sample (JP 2004-301646 A). These methods show some effects on inhibition of non-specific binding, but the effects are still insufficient.

Furthermore, countermeasures to background signals in binding between a biotin-binding protein and biotin have been proposed, for example: a method involving contact of a sample with a solid phase to which inactivated (strept)avidin is bound and then contact with a solid phase to which active (strept)avidin is bound (JP H08-114590 A); removal of interference by crosslinking, fragmentation, or coupling with, for example, BSA of (strept)avidin molecules or by inactivation of (strept)avidin molecules through saturation with biotin or modification of the biotin-binding active sites (JP H09-510289 A); and an interference-removing agent containing a mutant protein of (strept)avidin having a reduced binding affinity to biotin by genetic engineering (JP H10-28589 A).

The following examples are also disclosed: a method involving binding of a biotinylated substance to an avidin-bound solid phase, and then contact with a conjugate of polyethylene glycol and biotin (JP H11-211727 A); and a method of putting a biotin-containing solution into contact with a solid phase (JP 2002-48794 A).

Unfortunately, these technologies are still insufficient for a reduction in non-specific binding and still have problems in practical use, such as complicated procedure, complicated preparation of agents, nonuniform agents, and complicated preparation of a recombinant protein (since avidin and streptavidin are produced in E. coli, usually, in an inactive inclusion form, restatement thereof under appropriate conditions is needed).

### Biotin-binding protein monomer

The tetramers of avidin and streptavidin are very stable both in biotin-free and biotin-binding states. It is known that the tetramers are not dissociated into smaller subunits even in 6 M urea or 6 M guanidine hydrochloride (Kurzan et al., J. Biol. Chem., (1991), 266: 14470-14477). Thus, application of monomer derived from native biotin-binding protein is unknown, and the monomer itself cannot be readily prepared. Accordingly, the monomers have received little attention.

In contrast, monomer proteins of avidin or streptavidin having mutation in the amino acid sequences have been produced.

Avidin, streptavidin, or tamavidin very strongly binds to biotin, and the binding is irreversible and is hardly dissociated. Because of this strong binding, the native avidin, streptavidin, and tamavidin cannot be directly applied to a technical field that requires reversible binding necessary for purifying biotinylated biological molecules, such as affinity chromatography. In order to solve this problem, in avidin and streptavidin, an example of reducing the affinity to biotin through mutation of the amino acid involved in the interaction between subunits of each protein to produce a mutant protein has been reported. Some of these mutant proteins are monomer proteins (Laitinen et al., (2001), J. Biol. Chem., 276: 8219-8224; Wu and Wong, (2005), J. Biol. Chem., 280: 23225-23231).

In avidin and streptavidin, each subunit has one biotin-binding site. In order to form a complete biotin-binding pocket, the amino acid residue present in the adjacent subunit is important. Accordingly, it is believed that monomers cannot form a complete biotin-binding pocket, resulting in a reduction in the affinity to biotin.

In order to apply a biotin-binding protein, such as avidin and streptavidin, to a technical field that requires reversible binding, such as affinity chromatography, a possible goal is to increase the dissociation constant (KD) to about 10⁻⁷ (M). Though it depends on circumstance, in general, dissociation constant less than this level leads to high biotin-binding ability that precludes efficient dissociation of a desired biotinylated substance, while a dissociation constant higher than this level leads to low biotin-binding ability that precludes sufficient binding of a desired biotinylated substance (Wu and Wong, (2006), Protein Expr. Purif., 46: 268-27). In actually produced mutant proteins, the monomers all have a biotin-binding ability of about 10⁻⁹ to 10⁻⁶ (M) (Qureshi et al., (2001), J. Biol. Chem., 276: 46422-46428). All of these monomers are not yet applied to practical use.

### CITATION LIST

### PATENT LITERATURE

Patent Literature 1: WO02/072817
Patent Literature 2: JP H04-236353 A
Patent Literature 3: JP S59-99257 A
Patent Literature 4: JP H08-43392 A
Patent Literature 5: JP 2004-301646 A
Patent Literature 6: JP H08-114590 A
Patent Literature 7: JP H09-510289 A
Patent Literature 8: JP H10-28589 A
Patent Literature 9: JP H11-211727 A
Patent Literature 10: JP 2002-48794 A

### NON-PATENT LITERATURE

Non-Patent Literature 1: Kurzan et al., (1991), J. Biol. Chem., 266: 14470-14477
Non-Patent Literature 2: Laitinen et al., (2001), J. Biol. Chem., 276: 8219-8224
Non-Patent Literature 3: Wu and Wong, (2005), J. Biol. Chem., 280: 23225-23231
Non-Patent Literature 4: Wu and Wong, (2006), Protein Expr. Purif., 46: 268-27
Non-Patent Literature 5: Qureshi et al., (2001), J. Biol. Chem., 276: 46422-46428

### SUMMARY OF INVENTION

### TECHNICAL PROBLEM

It is an object of the present invention to provide a method of inhibiting non-specific binding in a step of detecting a substance in a biological sample, the method comprising:
bringing a biotin-binding protein monomer not substantially having biotin-binding ability into contact with the biological sample, wherein
in the step of detecting, a substance capable of specifically binding to the substance to be detected is immobilized to a carrier through binding between biotin and the biotin-binding protein before or after binding of the substance to be detected to the substance capable of specifically binding to the substance to be detected.

It is another object of the present invention to provide a novel non-specific binding-inhibiting agent for use in the method.

It is a further object of the present invention to provide a biotin-binding protein monomer that can be used as the non-specific binding-inhibiting agent.

### SOLUTION TO PROBLEM

The present inventors have found that there are a certain number of serum samples in which non-specific interaction cannot be sufficiently prevented in a detection system using a biotin-binding protein, even if a known method is used. Accordingly, the inventors have diligently studied and have found that in a detection or measurement system utilizing a biotin-binding protein, non-specific binding is significantly decreased by adding a monomerized biotin-binding protein to the system, even in serum samples in which non-specific interaction cannot be sufficiently prevented by a known method, and have arrived at the present invention.

In conventional immunoassay using (strept)avidin having reduced biotin-binding activity as a non-specific binding-inhibiting agent, the (strept)avidin has been required to have a spatial structure that is not significantly different from that of natural (strept)avidin except that the biotin-binding ability has been modified. It has been believed that such a mutant protein preferably has high immunological cross-reactivity with a natural polypeptide and is also desirably capable of forming a dimer or tetramer (JP H10-28589 A).

However, the inventors have surprisingly found, in contrary to the common technical knowledge in the art, that the effect of reducing non-specific binding by a monomerized biotin-binding protein is significantly higher than that of the biotin-binding protein in a tetramer form.

Based on the findings described above, the present invention provides a method of effectively inhibiting non-specific binding and an agent for use in the method in a system of detecting or measuring a substance to be detected in a sample using a substance capable of specifically binding to the substance to be detected, wherein the substance is immobilized to a carrier through binding between biotin and a biotin-binding protein.

The present invention includes the following nonlimiting embodiments.

### [Embodiment 1]

A method of inhibiting non-specific binding in a step of detecting a substance in a biological sample, the method comprising:
bringing a biotin-binding protein monomer not substantially having biotin-binding ability into contact with the biological sample, wherein
in the step of detecting, a substance capable of specifically binding to the substance to be detected is immobilized to a carrier through binding between biotin and the biotin-binding protein before or after binding of the substance to be detected to the substance capable of specifically binding to the substance to be detected.

### [Embodiment 2]

The method of inhibiting according to Embodiment 1, wherein the biotin-binding protein monomer has a molecular weight of 5 to 20 kDa.

### [Embodiment 3]

The method of inhibiting according to Embodiment 1 or 2, wherein the biotin-binding protein monomer is non-aggregatable.

### [Embodiment 4]

The method of inhibiting according to any one of Embodiments 1 to 3, wherein the biotin-binding protein monomer is non-aggregatable in the absence of surfactants or at a refrigeration temperature of 2°C to 10°C.

### [Embodiment 5]

The method of inhibiting according to any one of Embodiments 1 to 4, wherein the biotin-binding protein monomer is prepared by treating a biotin-binding protein with heat or a chemical, or prepared by modifying one or more amino acid residues in the amino acid sequence of a biotin-binding protein.

### [Embodiment 6]

The method of inhibiting according to any one of Embodiments 1 to 4, wherein the biotin-binding protein monomer is prepared by treating a biotin-binding protein with heat.

### [Embodiment 7]

The method of inhibiting according to any one of Embodiments 1 to 6, wherein the biotin-binding protein is selected from the group consisting of tamavidin 2, tamavidin 1, streptavidin, and avidin.

### [Embodiment 8]

The method of inhibiting according to any one of Embodiments 1 to 7, wherein the biotin-binding protein from which the monomer is derived is the same species as that of the biotin-binding protein constituting binding between biotin and the biotin-binding protein for immobilizing the substance capable of specifically binding to the substance to be detected to the carrier.

### [Embodiment 9]

The method of inhibiting according to any one of Embodiments 1 to 8, wherein the substance capable of specifically binding to the substance to be detected used in the step of detecting is immobilized to the carrier through binding between biotin and the biotin-binding protein by any of the following A) to C):
A) binding a biotinylated substance capable of specifically binding to the substance to be detected to the biotin-binding protein immobilized to the carrier;
B) binding the biotin-binding protein to biotin immobilized to the carrier and then binding a biotinylated substance capable of specifically binding to the substance to be detected thereto; or
C) binding a fusion protein composed of the biotin-binding protein and the substance capable of specifically binding to the substance to be detected, to biotin immobilized to the carrier.

### [Embodiment 10]

An agent for inhibiting non-specific binding in a step of detecting a substance in a biological sample, the agent comprising:
a biotin-binding protein monomer not substantially having biotin-binding ability, wherein
in the step of detecting, a substance capable of specifically binding to the substance to be detected is immobilized to a carrier through binding between biotin and a biotin-binding protein before or after binding of the substance to be detected to the substance capable of specifically binding to the substance to be detected.

### [Embodiment 11]

The agent according to Embodiment 10, wherein the biotin-binding protein monomer has a molecular weight of 5 to 20 kDa.

### [Embodiment 12]

The agent according to Embodiment 10 or 11, wherein the biotin-binding protein monomer is non-aggregatable.

### [Embodiment 13]

The agent according to any one of Embodiments 10 to 12, wherein the biotin-binding protein monomer is non-aggregatable in the absence of surfactants or at a refrigeration temperature of 2°C to 10°C.

### [Embodiment 14]

The agent according to any one of Embodiments 10 to 13, wherein the biotin-binding protein monomer is prepared by treating a biotin-binding protein with heat.

### [Embodiment 15]

The agent according to any one of Embodiments 10 to 14, wherein the biotin-binding protein is selected from the group of consisting of tamavidin 2, tamavidin 1, streptavidin, and avidin.

### [Embodiment 16]

A biotin-binding protein monomer having the following properties i) to iii):
i) not substantially having biotin-binding ability;
ii) having a molecular weight of 5 to 20 kDa; and
iii) not aggregating.

### [Embodiment 17]

The monomer according to Embodiment 16 comprising an amino acid sequence selected from the group consisting of:
i) the amino acid sequence of SEQ ID NO: 2;
ii) the amino acid sequence of SEQ ID NO: 4;
iii) the amino acid sequence of SEQ ID NO: 6;
iv) the amino acid sequence of SEQ ID NO: 8;
v) amino acid sequences having deletion, substitution, insertion, and/or addition of one or several amino acids in the amino acid sequence of SEQ ID NO: 2, 4, 6, or 8;
vi) amino acid sequences having at least 80% identity with the amino acid sequence of SEQ ID NO: 2, 4, 6, or 8;
vii) amino acid sequences encoded by nucleic acids consisting of the nucleotide sequence of SEQ ID NO: 1, 3, 5, or 7;
viii) amino acid sequences encoded by nucleic acids consisting of the nucleotide sequence having deletion, substitution, insertion, and/or addition of one or more nucleotides in the nucleotide sequence of SEQ ID NO: 1, 3, 5, or 7;
ix) amino acid sequences encoded by nucleic acids consisting of the nucleotide sequence having at least 80% identity with the nucleotide sequence of SEQ ID NO: 1, 3, 5, or 7; and
x) amino acid sequences encoded by nucleic acids hybridizable under stringent hybridization conditions with nucleic acids consisting of nucleotide sequences complementary to the nucleotide sequence of SEQ ID NO: 1, 3, 5, or 7.

### [Embodiment 18]

The biotin-binding protein monomer according to Embodiment 16 or 17, prepared by treating a biotin-binding protein with heat.

### [Embodiment 19]

A use of a biotin-binding protein monomer not substantially having biotin-binding ability for inhibiting non-specific binding in a step of detecting a substance in a biological sample.

### [Embodiment 20]

A kit for a method of inhibiting non-specific binding in a step of detecting a substance in a biological sample, the kit comprising a non-specific binding-inhibiting agent containing a biotin-binding protein monomer not substantially having biotin-binding ability.

### ADVANTAGEOUS EFFECTS OF INVENTION

The present invention enables inhibition of non-specific binding caused by background noises in detection of a substance to be detected present in a sample in a system immobilizing a substance capable of specifically binding to the substance to be detected to a carrier by utilizing binding between biotin and a biotin-binding protein. The non-specific binding-inhibiting agent of the present invention enables detection of a substance that is present in a very small quantity in a biological sample and is usually difficult to be detected.

### BRIEF DESCRIPTION OF DRAWINGS

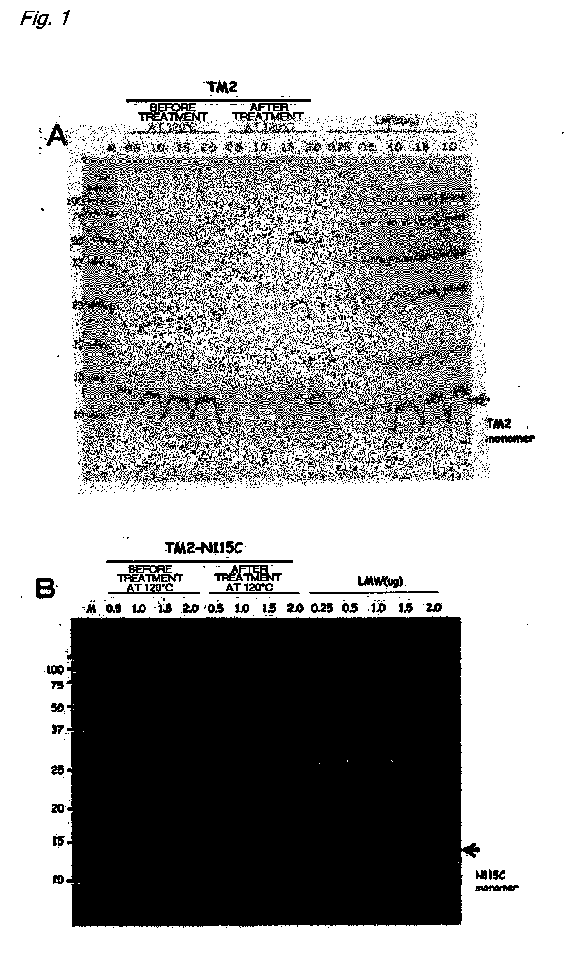

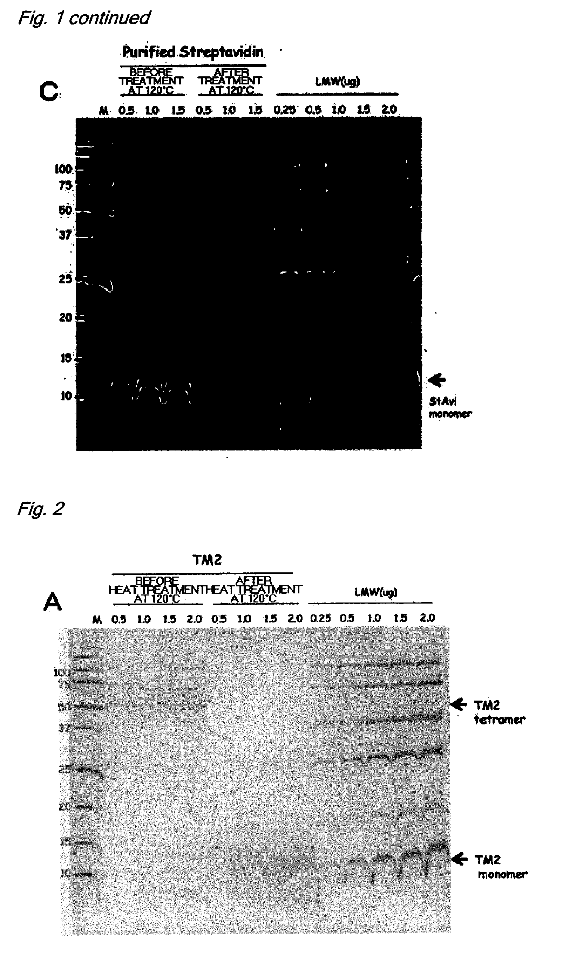

Fig. 1 illustrates purification and recovery of biotin-binding proteins by heat treatment, more specifically SDS-PAGE stained with CBB of samples roughly purified at 80°C (before the treatment at 120°C) and samples further treated at 120°C for 15 minutes. Tamavidin 2 (TM2) expressed in E. coli (Fig. 1A), thermostable tamavidin 2 (TM2-N115C) expressed in E. coli (Fig. 1B), and commercially available purified streptavidin (not roughly purified at 80°C) (Fig. 1 C) were used in the experiment.

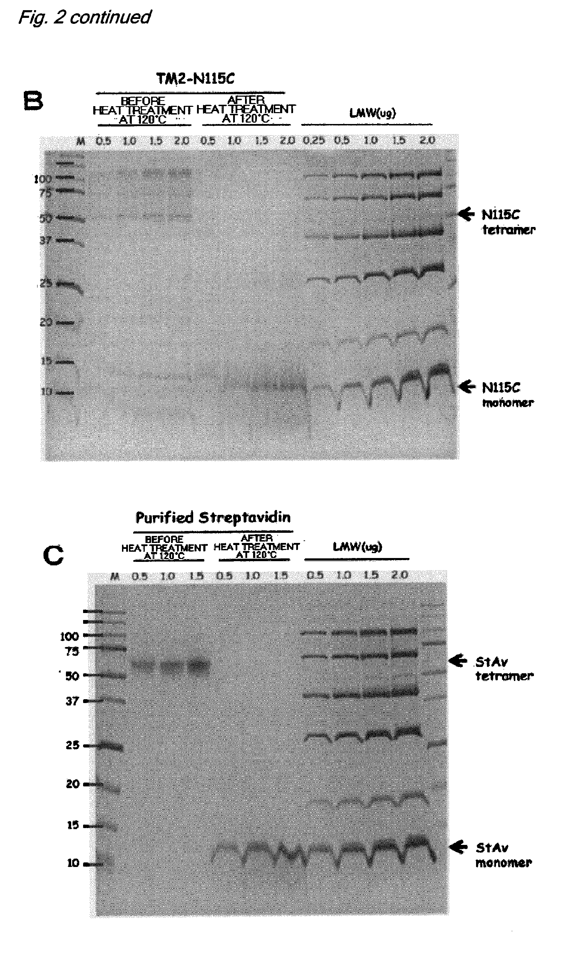

Fig. 2 illustrates monomerization of biotin-binding proteins by heat treatment, more specifically SDS-PAGE stained CBB with of samples roughly purified at 80°C (before the treatment at 120°C) and samples further treated at 120°C for 15 minutes. Tamavidin 2 (TM2) expressed in E. coli (Fig. 2A), thermostable tamavidin 2 (TM2-N115C) expressed in E. coli (Fig. 2B), and commercially available purified streptavidin (not roughly purified at 80°C) (Fig. 2C) were used in the experiment. In the SDS-PAGE, in order to investigate assembling of the subunits of each protein, a sample buffer not containing DTT was used without heat treatment before electrophoresis.

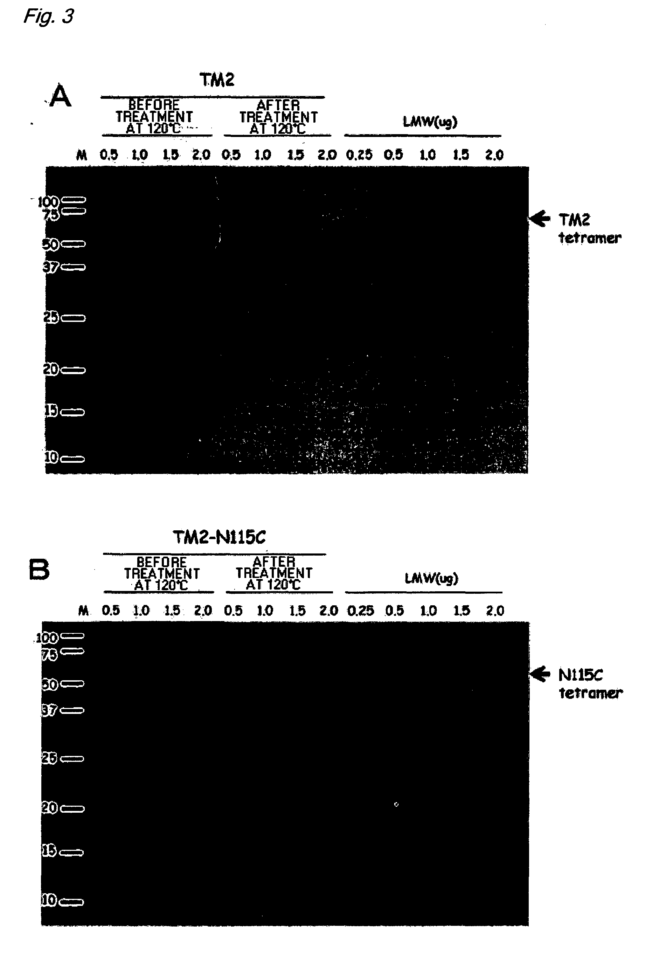

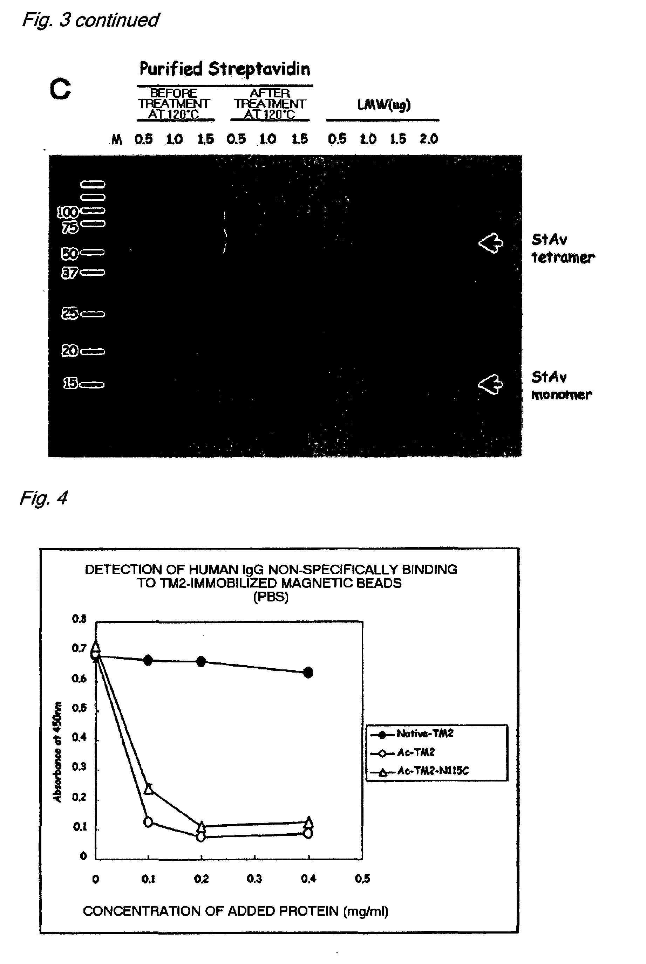

Fig. 3 illustrates inactivation of biotin-binding proteins by heat treatment, more specifically SDS-PAGE (used a sample buffer not containing DTT without heat treatment before electrophoresis), transferred to a PVDF membrane, and stained with biotinylated horseradish peroxidase of samples roughly purified at 80°C (before the treatment at 120°C) and samples further treated at 120°C for 15 minutes. Tamavidin 2 (TM2) expressed in E. coli (Fig. 3A), thermostable tamavidin 2 (TM2-N115C) expressed in E. coli (Fig. 3B), and commercially available purified streptavidin (not roughly purified at 80°C) (Fig. 3C) were used in the experiment.

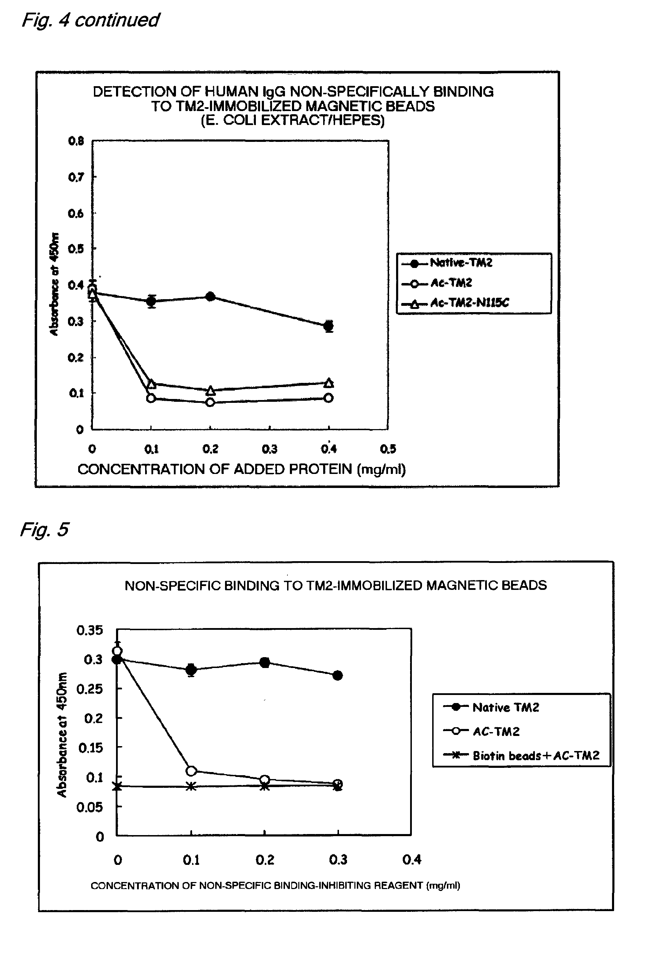

Fig. 4 includes graphs illustrating the results of measurement of non-specific binding to TM2-immobilized magnetic beads using a serum #R168334 (purchased from Kohjin Bio Co., Ltd.) and illustrating the effects of inhibiting non-specific binding of human IgG in serum by an inactivated biotin-binding protein monomer fraction, wherein the serum diluent used was 2% BSA/PBS (the left graph) or 2% BSA/E. coli extract/HEPES (the right graph); and the non-specific binding-inhibiting agent used was native TM2 (untreated purified TM2 tetramer), AC-TM2 (TM2 monomer prepared by treating TM2-expressing E. coli extract at 80°C and further treating the extract at 120°C for 15 min in an autoclave), or AC-TM2-N115C (TM2-N115C monomer prepared by treating TM2-N115C-expressing E. coli extract at 80°C and further treating the extract at 120°C for 15 min in an autoclave).

In the left and right graphs of Fig. 4, black circles, white circles, and white triangles represent the results of native TM2, AC-TM2, and AC-TM2-N115C, respectively.

Fig. 5 is a graph illustrating the results of measurement of non-specific binding to TM2-immobilized magnetic beads or to biotinylated magnetic beads using a serum #R168334, wherein the serum diluent used was 2% BSA/PBS; and the non-specific binding-inhibiting agent used was native TM2 or AC-TM2.

In Fig. 5, black circles and white circles represent the results of native TM2 and AC-TM2, respectively; and the symbol x indicates the results of a system to which AC-TM2 was added to biotinylated magnetic beads as a non-specific binding-inhibiting agent.

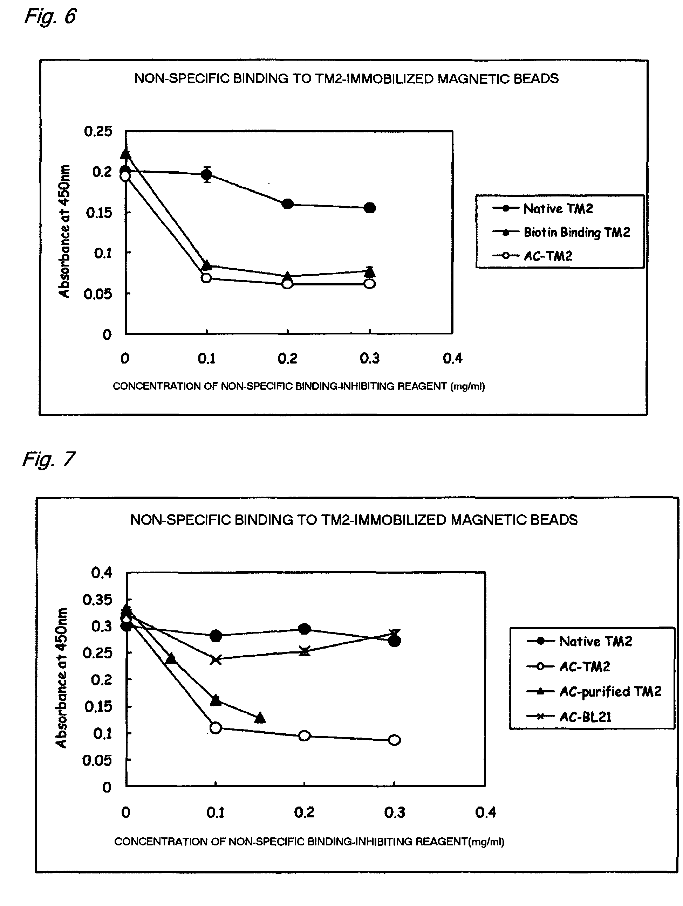

Fig. 6 illustrates the effects of inhibiting non-specific binding by biotin binding TM2, specifically, is a graph representing the results of measurement of non-specific binding to TM2-immobilized magnetic beads using a serum #R168334, wherein the serum diluent used was 2% BSA/E. coli crude extract/HEPES; and the non-specific binding-inhibiting agent used was native TM2, biotin binding TM2, or AC-TM2.

In Fig. 6, black circles, white circles, and black triangles represent the results of native TM2, AC-TM2, and biotin binding TM2, respectively.

Fig. 7 illustrates the effects of inhibiting non-specific binding by purified TM2 inactivated and monomerized by autoclave treatment, specifically, is a graph illustrating the results of measurement of non-specific binding to TM2-immobilized magnetic beads using a serum #R168334, wherein the serum diluent used was 2% BSA/PBS; and the non-specific binding-inhibiting agent used was native TM2, AC-TM2, AC-purified TM2 (prepared by heat treatment of purified TM2 at 120°C for 15 min in an autoclave), or AC-BL21 (prepared by treating an extract of E. coli BL21 cell line at 80°C and further treating the extract at 120°C for 15 min in an autoclave).

In Fig. 7, black circles, white circles, black triangles, and symbol x represent the results of native TM2, AC-TM2, AC-purified TM2, and AC-BL21, respectively.

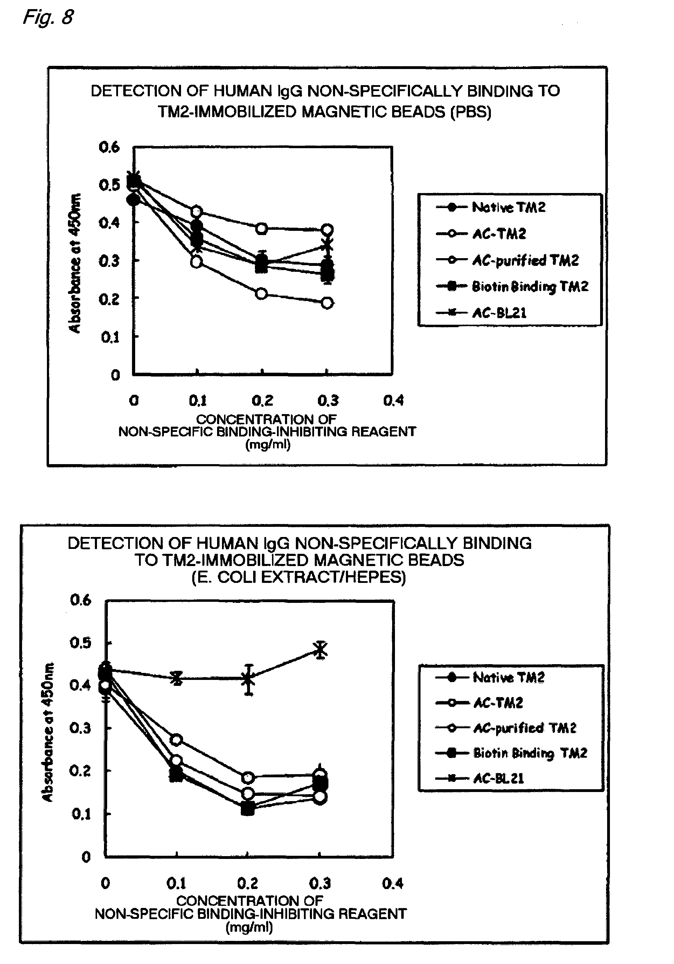

Fig. 8 illustrates non-specific binding to TM2-immobilized magnetic beads and effects of non-specific binding-inhibiting agents in a serum #R168216, specifically, includes graphs illustrating the results of measurement of non-specific binding to TM2-immobilized magnetic beads using the serum #R168216, wherein the serum diluent used was 2% BSA/PBS (the left graph) or 2% BSA/HEPES/E. coli crude extract (the right graph); and the non-specific binding-inhibiting agent used was native TM2, biotin binding TM2, AC-TM2, and AC-BL21.

In the left and right graphs of Fig. 8, black circles, white circles, gray circles, black squares, and symbol x represent the results of native TM2, AC-TM2, AC-purified TM2, biotin binding TM2, and AC-BL21, respectively.

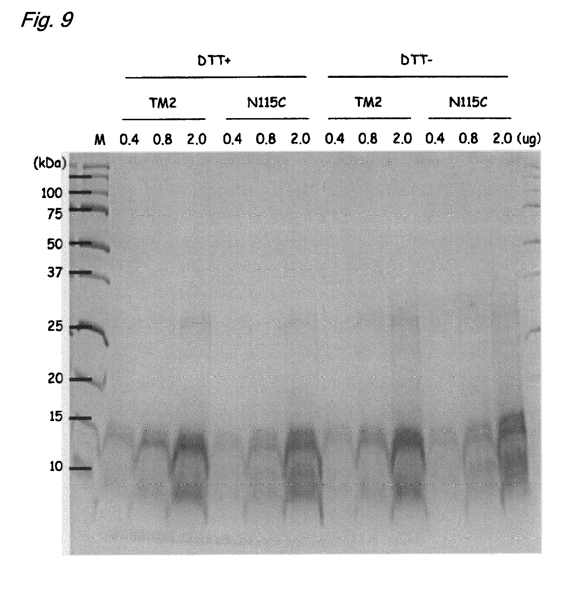

Fig. 9 illustrates the results of SDS-PAGE for investigating the molecular weights of a TM2 monomer and a TM2-N115C monomer reserved at 4°C for one month. The solutions of the TM2 monomer and the TM2-N115C reserved at 4°C for one month were each subjected to SDS-PAGE at amounts of 0.4, 0.8, and 2.0 µg/lane, followed by staining with CBB.

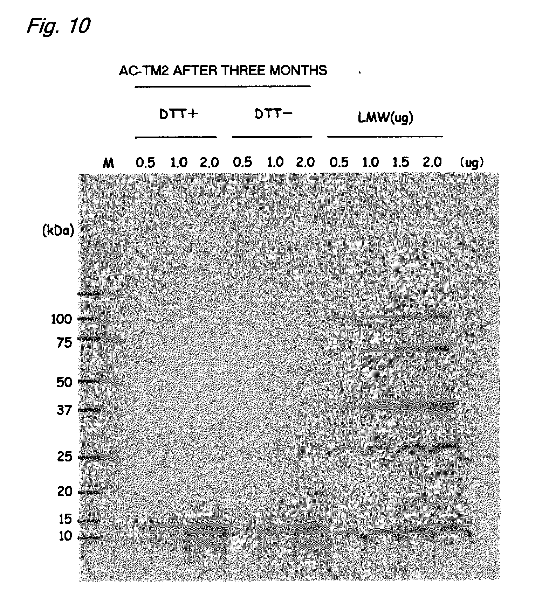

Fig. 10 illustrates the results of SDS-PAGE for investigating the molecular weight of a TM2 monomer reserved at 4°C for three months. A solution of the TM2 monomer reserved at 4°C for three months was subjected to SDS-PAGE at amounts of 0.5, 1.0, and 2.0 µg/lane, followed by staining with CBB. The SDS-PAGE was performed for a section (DTT+ in the photograph) using a common sample buffer (containing DTT) with heat treatment before the electrophoresis and for a section (DTT- in the photograph) using a DTT-free sample buffer without heat treatment before the electrophoresis for investigating assembling of the subunits of the protein.

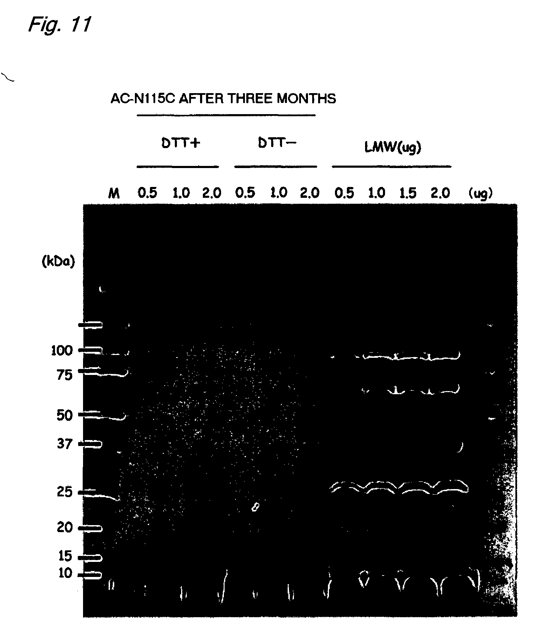

Fig. 11 illustrates the results of the molecular weight of a TM2-N115C monomer reserved at 4°C for three months by SDS-PAGE. A solution of the TM2-N115C monomer reserved at 4°C for three months was subjected to SDS-PAGE at amounts of 0.5, 1.0, and 2.0 µg/lane, followed by staining with CBB. The SDS-PAGE was performed for a section (DTT+ in the photograph) using a common sample buffer (containing DTT) with heat treatment before the electrophoresis and for a section (DTT- in the photograph) using a DTT-free sample buffer without heat treatment before the electrophoresis for investigating assembling of the subunits of the protein.

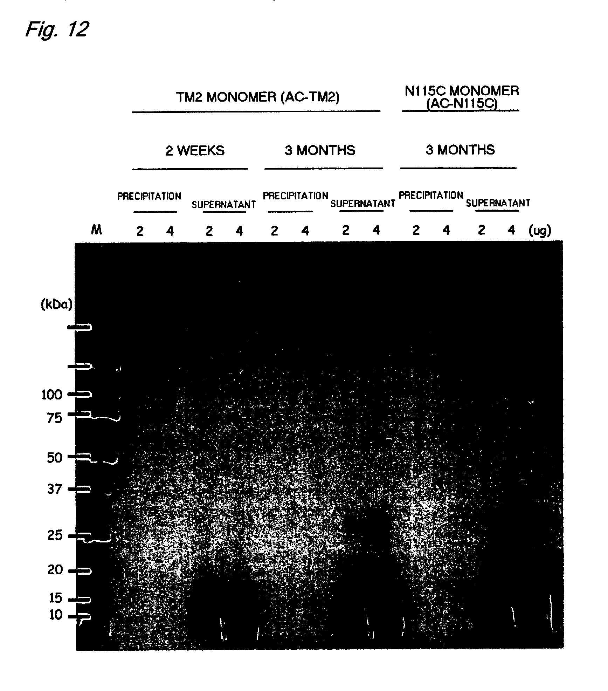

Fig. 12 illustrates the results of a test for investigating aggregation and precipitation after storage at 4°C, wherein solutions of a TM2 monomer and a TM2-N115C monomer reserved at 4°C for two weeks or three months were centrifuged, and the supernatant and precipitation fractions of each monomer were each subjected to SDS-PAGE at amounts of 2.0 and 4.0 µg/lane, followed by staining with CBB to detect the protein. The volumes of the precipitated fraction loaded were the same respectively as those in 2.0 and 4.0 µg/lane of the supernatant fraction.

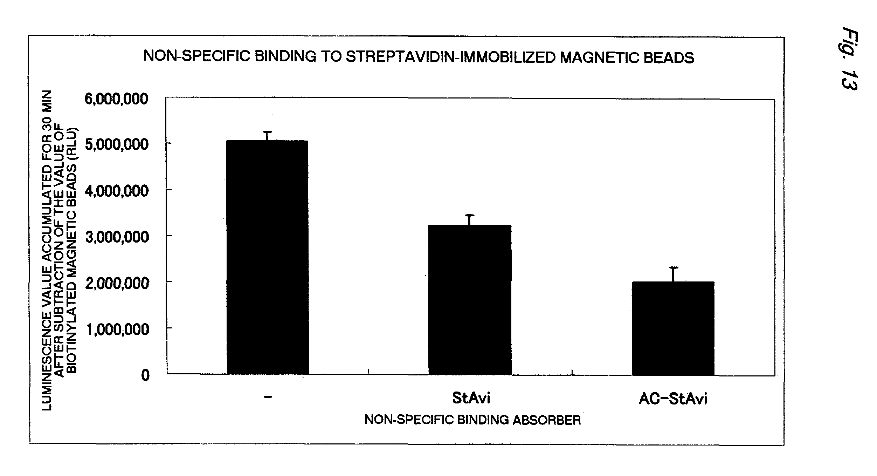

Fig. 13 illustrates the results of inhibiting non-specific binding by streptavidin inactivated and monomerized by heat treatment. The graph illustrates the value obtained by subtracting the measured value in biotinylated magnetic beads from the measured value in streptavidin-immobilized magnetic beads as the amount of IgG bound to streptavidin. "StAvi" and "AC-StAvi" respectively represent the results of streptavidin (tetramer) and streptavidin (monomer) inactivated by heat treatment used as non-specific binding blockers; and the symbol "-" represents the results of samples containing no additives.

### DESCRIPTION OF EMBODIMENTS

Embodiments for implementing the present invention will now be described.

### I. Method of inhibiting non-specific binding in step of detecting a substance in a biological sample

The detection step in the present invention utilizes immobilization of a substance capable of specifically binding to a substance to be detected to a carrier through binding between biotin and a biotin-binding protein, before or after binding of the substance to be detected to the substance capable of specifically binding to the substance to be detected. The method of inhibiting non-specific binding of the present invention comprises bringing a monomer of a biotin-binding protein not substantially having biotin-binding ability into contact with the biological sample.

### Sample

In the present invention, the sample usable in the detection step may be any sample probably containing a substance to be detected and is preferably a biological sample. Examples of the sample include cells and tissues collected from organisms and fragments thereof, for example, body fluids, more preferably, blood, serum, plasma, cerebrospinal fluid, saliva, sweat, urine, tear, lymph fluid, and mother's milk.

These body fluids may be used after dilution as needed. The dilution factor is, but not limited to, generally in the range of about 2 to about 10000 fold, preferably about 100 to 1000 fold. The solution for dilution may be any buffer solution, which may contain any proper blocking agent. Preferred blocking agents have high inhibitory effect on non-specific binding, and can be selected from blocking agents well-known to persons skilled in the art, such as BSA and casein.

The substance to be detected in the present invention may be any substance that is desired to be detected or measured in a sample, and preferred examples thereof include proteins such as antibodies and antigens and their fragments, peptides, nucleic acids, carbohydrates, and glycolipids.

### Substance capable of specifically binding to a substance to be detected

In the present invention, examples of the substance that specifically binds to a substance to be detected, i.e., the substance capable of specifically binding to a substance to be detected (which may also be referred to as "specific binding substance" throughout the specification) include, but not limited to, proteins, nucleic acids, and glycolipids. In one non-limiting embodiment of the present invention, for example, one of an antigen and an antibody, a ligand, such as a hormone, and a receptor, a lectin and a saccharide, or complementary bindings of a nucleic acid in a sample to be tested is selectively analyzed by means of the ability to form a specific complex with the other.

More specifically, examples of the protein include, but not limited to, antibodies, antigenic proteins, lectins, peptides, protein A, protein G, protein L, receptors, and enzymatic proteins. Examples of the antibody include IgG and antibody fragments containing antigen-binding sites, such as scFv and Fab. Examples of the antigenic protein include proteins derived from viruses such as hepatitis B and C viruses, HIVs, influenza viruses, and herpes viruses; proteins derived from bacteria such as Helicobacter pylori; tumor markers such as CEA and PSA; and sex hormones. The lectin is a saccharide-binding protein, and examples thereof include monosaccharide specific lectins, such as mannose specific lectin, GalNAc specific lectin, GlcNAc specific lectin, fucose specific lectin, and sialic acid specific lectin; and oligosaccharide specific lectins. Further examples include DNA/RNA binding proteins. Examples of the peptide include those composed of 2 to 100 amino acids, preferably 4 to 50 amino acids, and more preferably 6 to 30 amino acids, but not limited thereto.

Nonlimiting examples of the nucleic acid include DNAs, RNAs, and peptide nucleic acids (PNAs). Nonlimiting examples of the saccharide include monosaccharides, disaccharides, trisaccharides, oligosaccharides, and polysaccharides; and nonlimiting examples of the glycolipid include galactolipids, sulfolipids, and glycosphingolipids.

### Carrier to which substance capable of specifically binding to a substance to be detected is bound through binding between biotin and biotin-binding protein

The detection step in the present invention utilizes immobilization of a substance capable of specifically binding to a substance to be detected to a carrier through binding between biotin and a biotin-binding protein, before or after binding of the substance to be detected to the substance capable of specifically binding to the substance to be detected.

In the present invention, the "bond between biotin and a biotin-binding protein" may be referred to as "avidin-biotin bonding".

### i) Biotin

"Biotin" is a generic name of D-[(+)-cis-hexahydro-2-oxo-1H-thieno-(3,4)-imidazole-4-valeric acid]. It is one of the water-soluble vitamins categorized into a vitamin B group, and is also referred to as vitamin B₇, vitamin H, or coenzyme R. Biotin very strongly binds to avidin, one of the glycoproteins contained in albumen, so that its absorption is precluded. Thus, a large dose of uncooked albumen may cause biotin deficiency disease.

Throughout the specification, the term "biotin" includes iminobiotin (Hofmann et al., (1980), Proc. Natl. Acad. Sci. USA, 77: 4666-4668), desthiobiotin (Hirsch et al., (2002), Anal. Biochem., 308: 343-357), and biotin analogs such as biocytin and biotin sulfoxide, in addition to the biotin described above.

Systems using biotin-avidin (biotin-binding protein) complexes are widely used in the fields of biochemistry, molecular biology, tissue immunology, DNA analysis, and clinical assay. Also in the present invention, a specific binding substance is immobilized to a carrier through avidin-biotin bonding.

### ii) Biotin-binding protein

In the present invention, the specific binding substance may be immobilized to a carrier with any biotin-binding protein, and any protein known as a biotin-binding protein can be used.

Any protein that strongly binds to biotin can be preferably used as the biotin-binding protein, and examples thereof include avidin, streptavidin, neutravidin, AVR protein (Biochem. J., (2002), 363: 609-617), bradavidin (J. Biol. Chem., (2005), 280: 13250-13255), rhizavidin (Biochem. J., (2007), 405: 397-405), tamavidin (WO2002/072817), and mutants thereof. The dissociation constant (KD) with biotin of such a biotin-binding protein is preferably 10⁻⁸ M or less, more preferably 10⁻¹⁰ M or less, and most preferably 10⁻¹² M or less.

The biotin-binding protein is, without any limitation, preferably selected from the group consisting of tamavidin 2, tamavidin 1, streptavidin, and avidin.

The biotin-binding activity of a biotin-binding protein can be measured by a known method, e.g., may be measured by the process using fluorescent biotin as described in Kada, et al., (Biochim. Biophys. Acta, 1427: 33-43 (1999)). This process is an assay system utilizing a property that the fluorescent intensity of fluorescent biotin is quenched by binding of the fluorescent biotin to the biotin-binding site of a biotin-binding protein. Alternatively, the biotin-binding activity of a biotin-binding protein also can be evaluated with a sensor that can measure the binding between the protein and biotin, such as a biosensor based on surface plasmon resonance principle. Alternatively, the biotin-binding activity of a biotin-binding protein can be evaluated by immobilizing the biotin-binding protein to a carrier such as a film or microbeads, reacting a biotinylated enzyme such as biotinylated horse radish peroxidase (HRP) with the biotin-binding protein, and measuring the enzyme activity after washing.

### Tamavidin

Particularly preferred biotin-binding proteins for immobilizing a specific binding substance to a carrier are tamavidin and mutants thereof. Tamavidin is a biotin-binding protein discovered in an edible mushroom, Pleurotus cornucopiae (WO2002/072817, Takakura et al., (2009), FEBS J., 276: 1383-1397). An example of the mutants of tamavidin is tamavidin exhibiting high binding capability and low non-specific binding characteristics (WO2010/018859).

The term "tamavidin" in the present invention refers to tamavidin 1 (TM1: amino acid sequence of SEQ ID NO: 4, nucleotide sequence of SEQ ID NO: 3), tamavidin 2 (TM2: amino acid sequence of SEQ ID NO: 2, nucleotide sequence of SEQ ID NO: 1), or a mutant thereof. Specifically, tamavidin of the present invention may be typically a protein comprising the amino acid sequence of SEQ ID NO: 4 or SEQ ID NO: 2, or a protein encoded by a nucleic acid comprising the nucleotide sequence of SEQ ID NO: 3 or SEQ ID NO: 1.

Alternatively, tamavidin of the present invention may be a protein that is a mutant of a protein comprising the amino acid sequence of SEQ ID NO: 4 or SEQ ID NO: 2 or a protein encoded by a nucleic acid comprising the nucleotide sequence of SEQ ID NO: 3 or SEQ ID NO: 1 and having biotin-binding activity similar to that of tamavidin 1 or 2 or high binding capability and low non-specific binding characteristics. Throughout the specification, tamavidin 1, tamavidin 2, and mutants thereof may be collectively referred to as tamavidin.

The mutant of tamavidin 1 or 2 may be a protein comprising an amino acid sequence having deletion, substitution, insertion, and/or addition of one or more amino acids in the amino acid sequence of SEQ ID NO: 4 or 2 and having biotin-binding activity similar to that of tamavidin 1 or 2.

Specifically, the mutant is a protein consisting of an amino acid sequence having deletion, substitution, insertion, and/or addition of one or more (preferably one to several (e.g., 1 to 40, 1 to 30, 1 to 20, or 1 to 15, and more preferably 10, 9, 8, 7, 6, 5, 4, 3, 2, or 1) amino acids in the amino acid sequence of SEQ ID NO: 4 or 2 and having the activities of the present invention described above. Throughout the specification, "amino acid sequence having deletion, substitution, insertion, and/or addition" means that the amino acid sequence has deletion, substitution, insertion, and/or addition of one or more amino acid residues at arbitrary one or more positions. Two or more of deletion, substitution, insertion, and addition may simultaneously occur, and a smaller number of deletion, substitution, insertion, and/or addition is generally preferred.

Among them, substitution is preferably conservative substitution, which means the replacement of a certain amino acid residue by another residue having similar physical and chemical characteristics. It may be any substitution that does not substantially alter the structural characteristics of the original sequence. Nonlimiting examples of the conservative substitution include substitutions between amino acid residues containing aliphatic groups, such as mutual substitution between Ile, Val, Leu, and Ala; and substitutions between polar residues, such as mutual substitution between Lys and Arg, between Glu and Asp, and between Gln and Asn.

In non-conservative substitution, a member of one of these groups can be replaced by a member from another group. In order to maintain the biological function of the protein of the present invention in such a case, the hydropathic indices of amino acids (hydropathic amino acid indices) (Kyte, et al., J. Mol. Biol., 157: 105-131 (1982)) are preferably considered. In the non-conservative substitution, amino acid substitutions can be accomplished on the basis of hydrophilicity.

A protein consisting of an amino acid sequence having deletion, substitution, or addition of one or more amino acids in the amino acid sequence of SEQ ID NO: 4 or 2 can be prepared according to techniques such as site-directed mutagenesis as described in, for example, "Molecular Cloning, A Laboratory Manual 3rd ed." (Cold Spring Harbor Press

(2001)). Throughout the specification, the term "one or more amino acids" refers to an amino acid or amino acids that can be deleted, substituted, inserted, and/or added by preferably site-specific mutagenesis.

The mutant of tamavidin 1 or 2 may also be a protein comprising an amino acid sequence having an amino acid identity of at least 80% or more, 85% or more, 90% or more, 95% or more, 96% or more, 97% or more, 98% or more, or 99% or more and more preferably 99.3% or more with the amino acid sequence of SEQ ID NO: 4 or 2 and having biotin-binding activity similar to that of tamavidin 1 or 2 or high binding capability and low non-specific binding characteristics.

Examples of the mutant of tamavidin also include the followings:

Proteins comprising amino acid sequences encoded by nucleic acids consisting of nucleotide sequences having deletion, substitution, insertion, and/or addition of one or more nucleotides in the nucleotide sequence of SEQ ID NO: 3 or SEQ ID NO: 1 and having biotin-binding activity similar to that of tamavidin 1 or 2 or high binding capability and low non-specific binding characteristics;

Proteins comprising amino acid sequences encoded by nucleic acids consisting of nucleotide sequences having an identity of 80% or more with the nucleotide sequence of SEQ ID NO: 3 or SEQ ID NO: 1 and having biotin-binding activity similar to that of tamavidin 1 or 2 or high binding capability and low non-specific binding characteristics; and

Proteins encoded by nucleic acids hybridizable under stringent hybridization conditions with nucleic acids consisting of nucleotide sequences complementary to the nucleotide sequence of SEQ ID NO: 3 or SEQ ID NO: 1 and having biotin-binding activity similar to that of tamavidin 1 or 2 or high binding capability and low non-specific binding characteristics.

The "deletion, substitution, insertion, and/or addition of one or more nucleotides" refers to a nucleotide or nucleotides that can be deleted, substituted, inserted, and/or added by preferably site-specific mutagenesis, specifically, a nucleotide sequence having deletion, substitution, insertion, and/or addition of one or more (preferably one to several (e.g., 1 to 120, 1 to 90, 1 to 60, or 1 to 45 and more preferably 30, 27, 24, 21, 18, 15, 12, 9, 6, or 1 to 3)) nucleotides in the nucleotide sequence of SEQ ID NO: 3 or 1.

The percent identity between two amino acid sequences may be determined by visual inspection and mathematical calculation. Alternatively, the percent identity between two protein sequences may be determined through comparison of sequences using a GAP computer program available from the University of Wisconsin Genetics Computer Group (UWGCG) based on the algorithm by Needleman, S. B. and Wunsch, C. D. (J. Mol. Biol., 48: 443-453, 1970). Preferred default parameters of the GAP program include: (1) scoring matrix: blosum62 described in Henikoff, S. and Henikoff, J. G., (Proc. Natl. Acad. Sci. USA, 89: 10915-10919, 1992); (2) 12 gap weights; (3) 4 gap length weights; and (4) no penalty for terminal gaps.

Any other program used by persons skilled in the art may also be used for comparison of the sequences. The percent identity can be determined by, for example, comparison of sequences using a BLAST program described in Altschul et. al., (Nucl. Acids. Res., 25, pp. 3389-3402, 1997). This program is available from the websites of National Center for Biotechnology Information (NCBI) or DNA Data Bank of Japan (DDBJ) on the Internet. The conditions (parameters) for identity search by the BLAST program is described in detail on these sites. Although these parameters can be partly modified if necessary, search is generally carried out using the default values. Alternatively, the percent identity between two amino acid sequences may be determined using a program such as genetic information processing software GENETYX Ver. 7 (available from GENETYX CORPORATION) or FASTA algorithm, wherein search may be carried out using the default values.

The percent identity between two nucleotide sequences can be determined by visual inspection and mathematical calculation. Preferably, such comparison is carried out through comparison of sequences using a computer program. A particularly preferred computer program is a version 10.0 program "GAP", Wisconsin package of Genetics Computer Group (GCG, Madison, Wisconsin) (Devereux, et al., 1984, Nucl. Acids Res., 12: 387). The use of the "GAP" program enables comparison between two amino acid sequences and comparison between a nucleotide sequence and an amino acid sequence, in addition to comparison of two nucleotide sequences. The preferred default parameters for the "GAP" program include: (1) the GCG implementation of a unary comparison matrix (containing a value of 1 for identities and 0 for non-identities) for nucleotides, and the weighted amino acid comparison matrix of Gribskov and Burgess, Nucl. Acids Res., 14: 6745, 1986, as described in Schwartz and Dayhoff, eds., "Atlas of Polypeptide Sequence and Structure", National Biomedical Research Foundation, pp. 353-358, 1979, or other comparable comparison matrices; (2) a penalty of 30 for each gap for amino acids and an additional penalty of 1 for each symbol in each gap, or a penalty of 50 for each gap for nucleotide sequences and an additional penalty of 3 for each symbol in each gap; (3) no penalty for end gaps; and (4) no maximum penalty for long gaps. Other sequence comparison programs used by those skilled in the art can also be used. For example, the BLASTN program version 2.2.7, which is available via the National Library of Medicine website: http://www.ncbi.nlm.nih.gov/blast/b12seq/bls.html, or the UW-BLAST 2.0 algorithm can be used. Setting of the standard default parameters for the UW-BLAST 2.0 is described at the following Internet site: http://blast.wustl.edu. In addition, the BLAST algorithm uses the BLOSUM62 amino acid scoring matrix, and optional parameters that can be used are as follows: (A) inclusion of a filter to mask segments of the query sequence having low compositional complexity (determined by the SEG program of Wootton and Federhen (Computers and Chemistry, 1993); also see Wootton and Federhen, 1996, "Analysis of compositionally biased regions in sequence databases", Methods Enzymol., 266: 544-71) or segments consisting of short-periodicity internal repeats (determined by the XNU program of Claverie and States (Computers and Chemistry, 1993)), and (B) a statistical significance threshold for reporting matches against database sequences or E-score (the expected probability of matches being found merely by chance, in accordance with the statistical model (Karlin and Altschul, 1990); if the statistical significance ascribed to a match is greater than the E-score threshold, the match will not be reported.); preferred E-score threshold values are 0.5, or in order of increasing preference, 0.25, 0.1, 0.05, 0.01, 0.001, 0.0001, 1e-5, 1e-10, 1e-15, 1e-20, 1e-25, 1e-30, 1e-40, 1e-50, 1e-75, or 1e-100.

The mutant of tamavidin may be a protein encoded by a nucleic acid comprising a nucleotide sequence hybridizable with the complementally strand of the nucleotide sequence of SEQ ID NO: 3 or 1 and having a binding activity similar to that of tamavidin.

Herein, the term "under stringent conditions" refers to that hybridization occurs under moderately or highly stringent conditions. Specifically, moderately stringent conditions can be readily determined by those having ordinary skill in the art, e.g., on the basis of the length of DNA. The basic conditions are set forth by Sambrook, et al., Molecular Cloning: A Laboratory Manual, 3rd edition, chapter 6, Cold Spring Harbor Laboratory Press, 2001 and include the use of a prewashing solution of, for example, 5 × SSC, 0.5% SDS, and 1.0 mM EDTA (pH 8.0), hybridization conditions of about 50% formamide, 2 to 6 × SSC, preferably 5 to 6 × SSC, and 0.5% SDS at about 42°C (or other similar hybridization solutions, such as Stark's solution, in about 50% formamide at about 42°C), and washing conditions of, for example, about 50°C to 68°C, 0.1 to 6 × SSC, and 0.1% SDS. Preferably, moderately stringent conditions include hybridization conditions (and washing conditions) of about 50°C, 6 × SSC, and 0.5% SDS. Highly stringent conditions can also be readily determined by those skilled in the art, e.g., depending on the length of DNA.

In general, highly stringent conditions include hybridization and/or washing at higher temperature and/or lower salt concentration (for example, hybridization in 6 to 0.2 × SSC, preferably 6 × SSC, more preferably 2 × SSC, more preferably 0.2 × SSC, or 0.1 × SSC containing about 0.5% SDS at about 65°C), compared to the moderately stringent conditions, and also include the hybridization conditions defined above with washing at approximately 65°C to 68°C, 0.2 to 0.1 × SSC, and 0.1% SDS. With the hybridization and washing buffer, SSPE (1 × SSPE is 0.15 M NaCl, 10 mM NaH₂PO₄, and 1.25 mM EDTA, pH 7.4) can be substituted for SSC (1 × SSC is 0.15 M NaCl and 15 mM sodium citrate). The washing is performed for about 15 min to 1 hr after the completion of the hybridization.

A commercially available hybridization kit including a non-radioactive probe can also be used. Specifically, hybridization with an ECL direct labeling & detection system (manufactured by Amersham) is available. For example, stringent hybridization conditions include hybridization with the hybridization buffer included in the kit to which a blocking agent and NaCl are added into concentrations of 5% (w/v) and 0.5 M, respectively, at 42°C for 4 hr and washing twice in 0.4% SDS, 0.5 × SSC at 55°C for 20 min and once in 2 × SSC at room temperature for 5 min.

The biotin-binding protein used for immobilizing a specific binding substance to a carrier preferably strongly binds to biotin as described above. Accordingly, it is preferred that the biotin-binding activity of a mutant of tamavidin 1 or 2 used for immobilizing a substance capable of specifically binding to a substance to be detected to a carrier be not significantly decreased compared to that of native tamavidin 1 or 2.

Accordingly, in the mutant of tamavidin 1 for immobilizing a specific binding substance to a carrier, preferably, N14, S18, Y34, S36, S78, W82, W98, W110, and D118 in the amino acid sequence of SEQ ID NO: 4 should not be modified. Note that the notation, for example, Y34 indicates the 34th tyrosine residue of the amino acid sequence of SEQ ID NO: 4. Alternatively, in the modification of such an amino acid, the amino acid is preferably modified to one having a similar property or structure. For example, asparagine (N14) is modified to glutamine (Q) or aspartic acid (D), preferably aspartic acid; serine (S18, S36, or S78) to threonine (T) or tyrosine (Y), preferably threonine; tyrosine (Y34) to serine (S), threonine (T), or phenylalanine (F), preferably phenylalanine; tryptophan (W82, W98, or W110) to phenylalanine (F); and aspartic acid (D118) to glutamic acid (E) or asparagine (N), preferably asparagine.

In the mutant of tamavidin 2 for immobilizing a specific binding substance to a carrier, preferably, four tryptophan residues (W69, W80, W96, and W108) in the amino acid sequence of SEQ ID NO: 2 are not modified. Alternatively, in the modification of such an amino acid, the amino acid is preferably modified to one having a similar property or structure, for example, phenylalanine(F). In addition, it is desirable that amino acid residues (N14, S18, Y34, S36, S76, T78, and D116) that probably interact directly with biotin are also not modified. Alternatively, in the modification of such an amino acid, the amino acid is preferably modified to one having a similar property or structure in order to maintain the binding with biotin. For example, asparagine (N14) is modified to glutamine (Q) or aspartic acid (D), preferably aspartic acid; aspartic acid (D40) to asparagine (N); serine (S18, S36, or S76) to threonine (T) or tyrosine (Y), preferably threonine; tyrosine (Y34) to serine (S), threonine (T), or phenylalanine (F), preferably phenylalanine; threonine (T78) to serine (S) or tyrosine (Y), preferably serine; and aspartic acid (D116) to glutamic acid (E) or asparagine (N), preferably asparagine.

Preferred tamavidin mutants in the present invention include the following mutants (WO2010/018859):

The tamavidin mutant is a modified biotin-binding protein comprising the amino acid sequence of SEQ ID NO: 2, an amino acid sequence having one to several amino acid mutations in this sequence, or an amino acid sequence having an identity of at least 80% with this sequence and showing biotin-binding activity, wherein one or more residues selected from the group consisting of:
1) the arginine residue at position 104 of SEQ ID NO: 2;
2) the lysine residue at position 141 of SEQ ID NO: 2;
3) the lysine residue at position 26 of SEQ ID NO: 2; and
4) the lysine residue at position 73 of SEQ ID NO: 2
are replaced with acidic or neutral amino acid residues.

More preferably, the tamavidin mutant is a modified biotin-binding protein selected from the group consisting of:
a modified biotin-binding protein (R104E-K141E) having replacement of the arginine residue at position 104 with a glutamic acid residue and replacement of the lysine residue at position 141 with a glutamic acid residue in SEQ ID NO: 2;
a modified biotin-binding protein (D40N-R104E) having replacement of the aspartic acid residue at position 40 with an asparagine residue and replacement of the arginine residue at position 104 with a glutamic acid residue in SEQ ID NO: 2;
a modified biotin-binding protein (D40N-K141E) having replacement of the aspartic acid residue at position 40 with an asparagine residue and replacement of the lysine residue at position 141 with a glutamic acid residue in SEQ ID NO: 2; and
a modified biotin-binding protein (D40N-R104E-K141E) having replacement of the aspartic acid residue at position 40 with an asparagine residue, replacement of the arginine residue at position 104 with a glutamic acid residue, and replacement of the lysine residue at position 141 with a glutamic acid residue in SEQ ID NO: 2.

### Streptavidin

Preferred biotin-binding proteins for immobilizing a specific binding substance to a carrier are streptavidin and its mutants. Streptavidin is an avidin-like protein derived from Streptomyces avidinii and does not have a sugar chain.

Streptavidin has an amino acid sequence of SEQ ID NO: 6 in the Sequence Listing. In this sequence, the first 24 amino acids are removed during maturation. Accordingly, the amino acid sequence of mature streptavidin protein is composed of D25 to Q183. The site, so-called core streptavidin, generated in nature is the sequence from A37 to S163, which is known to have biotin-binding activity (Sano et al., (1995), J. Biol. Chem., 270: 28204-28209).

The term "streptavidin" in the present invention refers to streptavidin or a mutant thereof. Specifically, streptavidin of the present invention may be typically a protein comprising the amino acid sequence from D25 to Q183 or from A37 to S163 in the amino acid sequence of SEQ ID NO: 6, or a protein encoded by a nucleic acid comprising the nucleotide sequence encoding from D25 to Q183 or from A37 to S163 in the nucleotide sequence of SEQ ID NO: 5.

The "sequence encoding from D25 to Q183 or from A37 to S163 in the nucleotide sequence of SEQ ID NO: 5" refers to the sequence from the nucleotide (g) at position 73 to the nucleotide (g) at position 552 and the sequence from the nucleotide (g) at position 109 to the nucleotide (c) at position 489 in the nucleotide sequence of SEQ ID NO: 5.

Alternatively, streptavidin of the present invention may be a mutant of a protein comprising the amino acid sequence from D25 to Q183 or from A37 to S 163 in the amino acid sequence of SEQ ID NO: 6 or a protein encoded by a nucleic acid comprising the nucleotide sequence encoding from D25 to Q183 or from A37 to S163 in the nucleotide sequence of SEQ ID NO: 5 and having biotin-binding activity similar to that of streptavidin.

The mutant of streptavidin may be a protein comprising an amino acid sequence having deletion, substitution, insertion, and/or addition of one or more amino acids in the amino acid sequence from D25 to Q183 or from A37 to S163 in the amino acid sequence of SEQ ID NO: 6 and having biotin-binding activity similar to that of streptavidin.

The mutant of streptavidin may also be a protein comprising an amino acid sequence having an amino acid identity of at least 80% or more, 85% or more, 90% or more, 95% or more, 96% or more, 97% or more, or 98% or more and more preferably 99% or more with the amino acid sequence from D25 to Q183 or from A37 to S163 in the amino acid sequence of SEQ ID NO: 6 and having biotin-binding activity similar to that of streptavidin.

Examples of the mutant of streptavidin also include the followings:

Proteins comprising amino acid sequences encoded by nucleic acids consisting of nucleotide sequences having deletion, substitution, insertion, and/or addition of one or more nucleotides in a nucleotide sequence comprising the sequence encoding from D25 to Q183 or from A37 to S163 in the amino acid sequence of SEQ ID NO: 6 in the nucleotide sequence of SEQ ID NO: 5 and having biotin-binding activity similar to that of streptavidin;

Proteins comprising amino acid sequences encoded by nucleic acids consisting of nucleotide sequences having an identity of 80% or more with a nucleotide sequence comprising the sequence encoding from D25 to Q183 or from A37 to S163 in the amino acid sequence of SEQ ID NO: 6 in the nucleotide sequence of SEQ ID NO: 5 and having biotin-binding activity similar to that of streptavidin; and

Proteins encoded by nucleic acids hybridizable under stringent hybridization conditions with nucleic acids consisting of nucleotide sequences complementary to nucleotide sequences comprising the sequence encoding from D25 to Q183 or from A37 to S163 in the amino acid sequence of SEQ ID NO: 6 in the nucleotide sequence of SEQ ID NO: 5 and having biotin-binding activity similar to that of streptavidin.

The meanings of "deletion, substitution, insertion, and/or addition of one or more amino acids", "deletion, substitution, insertion, and/or addition of one or more nucleotides", "percent identity", and "stringent hybridization conditions" are the same as those described for tamavidin.

The biotin-binding protein used for immobilizing a specific binding substance to a carrier preferably strongly binds to biotin as described above. Accordingly, it is preferred that the biotin-binding activity of a mutant of streptavidin used for immobilizing a substance capable of specifically binding to a substance to be detected to a carrier be not significantly decreased compared to that of native streptavidin.

Accordingly, in the mutant of streptavidin for immobilizing a specific binding substance to a carrier, preferably, N47, S51, Y67, S69, N73, W103, S112, T114, W116, W132, W144, and D152 in the amino acid sequence of SEQ ID NO: 6 are not modified. Note that the notation, for example, N47 indicates the 47th asparagine residue of the amino acid sequence of SEQ ID NO: 6. Alternatively, in the modification of such an amino acid, the amino acid is preferably modified to one having a similar property or structure.

### Avidin

Preferred biotin-binding proteins for immobilizing a specific binding substance to a carrier are avidin and its mutants. Avidin is a basic glycoprotein derived from albumen of Gallus gallus and strongly binds to biotin.

Avidin has an amino acid sequence of SEQ ID NO: 8 in the Sequence Listing. In this sequence, the first 24 amino acids are removed during maturation. Accordingly, the amino acid sequence of mature avidin protein is composed of A25 to E152.

The term "avidin" in the present invention refers to avidin or a mutant thereof. Specifically, avidin of the present invention may be typically a protein comprising the amino acid sequence from A25 to E152 in the amino acid sequence of SEQ ID NO: 8, or a protein encoded by a nucleic acid comprising the sequence encoding from A25 to E152 in the nucleotide sequence of SEQ ID NO: 7.

The "sequence encoding from A25 to E152 in the nucleotide sequence of SEQ ID NO: 7" refers to the sequence from the nucleotide (g) at position 73 to the nucleotide (a) at position 459 in the nucleotide sequence of SEQ ID NO: 7.

Alternatively, avidin of the present invention may be a mutant of a protein comprising the amino acid sequence from A25 to E152 in the amino acid sequence of SEQ ID NO: 8 or a protein encoded by a nucleic acid comprising the sequence encoding from A25 to E152 in the nucleotide sequence of SEQ ID NO: 7 and having biotin-binding activity similar to that of avidin.

The mutant of avidin may be a protein comprising an amino acid sequence having deletion, substitution, insertion, and/or addition of one or more amino acids in the amino acid sequence from A25 to E152 in the amino acid sequence of SEQ ID NO: 8 and having biotin-binding activity similar to that of avidin.

The mutant of avidin may also be a protein comprising an amino acid sequence having an amino acid identity of at least 80% or more, 85% or more, 90% or more, 95% or more, 96% or more, 97% or more, or 98% or more and more preferably 99% or more with the amino acid sequence from A25 to E152 in the amino acid sequence of SEQ ID NO: 8 and having biotin-binding activity similar to that of avidin.

Examples of the mutant of avidin also include the followings:

Proteins comprising amino acid sequences encoded by nucleic acids consisting of nucleotide sequences having deletion, substitution, insertion, and/or addition of one or more nucleotides in a nucleotide sequence comprising the sequence encoding from A25 to E152 in the nucleotide sequence of SEQ ID NO: 7 and having biotin-binding activity similar to that of avidin;

Proteins comprising amino acid sequences encoded by nucleic acids consisting of nucleotide sequences having an identity of 80% or more with a nucleotide sequence comprising the sequence encoding from A25 to E152 in the nucleotide sequence of SEQ ID NO: 7 and having biotin-binding activity similar to that of avidin; and

Proteins encoded by nucleic acids hybridizable under stringent hybridization conditions with nucleic acids consisting of nucleotide sequences complementary to nucleotide sequences comprising the sequence encoding from A25 to E152 in the nucleotide sequence of SEQ ID NO: 7 and having biotin-binding activity similar to that of avidin.

The meanings of "deletion, substitution, insertion, and/or addition of one or more amino acids", "deletion, substitution, insertion, and/or addition of one or more nucleotides", "percent identity", and "stringent hybridization conditions" are the same as those described for tamavidin.

The biotin-binding protein used for immobilizing a specific binding substance to a carrier preferably strongly binds to biotin as described above. Accordingly, it is preferred that the biotin-binding activity of a mutant of avidin used for immobilizing a substance capable of specifically binding to a substance to be detected to a carrier be not significantly decreased compared to that of native avidin.

Accordingly, in the mutant of avidin for immobilizing a specific binding substance to a carrier, preferably, N36, S40, Y57, T59, T62, A63, T64, W94, F96, S97, S99, T101, F103, W121, W134, and N142 in the amino acid sequence of SEQ ID NO: 8 are not modified. Note that the notation, for example, S40 indicates the 40th serine residue of the amino acid sequence of SEQ ID NO: 8. Alternatively, in the modification of such an amino acid, the amino acid is preferably modified one having a similar property or structure.

### iii) Carrier

Examples of materials for the solid carrier include, but not limited to, cellulose, Teflon, nitrocellulose, agarose, highly cross-linked spherical agarose, dextran, chitosan, polystyrene, polyacrylamide, polyesters, polycarbonates, polyamides, polypropylene, nylons, polydivinylidene difluoride, latex, polystyrene latex, silica, glass, glass fiber, gold, platinum, silver, copper, iron, stainless steel, ferrite, silicon wafers, polyethylene, polyethyleneimine, poly(lactic acid), resins, polysaccharides, proteins (such as albumin), carbon, and combination thereof. Preferred materials have a certain level of strength, a stable composition, and low non-specific binding characteristics.

Examples of the shape of the solid carrier include, but not limited to, microbeads, magnetic beads, thin films, capillary tubes, filters, plates, microplates, carbon nanotubes, and sensor chips. Flat solid carriers such as thin films and plates may be provided with pits, grooves, or filter bottoms, as is known in the art.

In an embodiment of the invention, microbeads may have a spherical diameter in the range of about 25 nm to about 1 mm. In a preferred embodiment, the beads have a diameter in the range of about 50 nm to about 10 µm.

If high detection sensitivity is desired for example, beads as described above can preferably be used as the solid carrier from the viewpoints of high contacting frequency between the substance to be detected and the substance that specifically binding to the substance to be detected and easiness in the cleaning process, in a nonlimiting embodiment.

### iv) Process of immobilizing a specific binding substance to a carrier

The immobilization of a substance capable of specifically binding to a substance to be detected to a carrier, which is utilized in the detection step of the present invention, may be achieved by any embodiment involving binding between biotin and a biotin-binding protein. The immobilization can be achieved by any of the following nonlimiting procedures A) to C):
A) Binding a biotinylated substance capable of specifically binding to a substance to be detected to a biotin-binding protein immobilized to the carrier;
B) Binding a biotin-binding protein to biotin immobilized to a carrier and then binding a biotinylated substance capable of specifically binding to a substance to be detected thereto; and
C) Binding a fusion protein composed of a biotin-binding protein and a substance capable of specifically binding to a substance to be detected, to biotin immobilized to a carrier.

The details will now be described.

### A) Process of binding a biotinylated substance capable of specifically binding to a substance to be detected to the biotin-binding protein immobilized to a carrier

In embodiment A), the biotin-binding protein can be directly bound to a carrier through hydrophobic bonding or covalent bonding. Alternatively, the biotin-binding protein may be directly bound and immobilized to a microplate such as NEW ELISA Plate kit (Sumitomo Bakelite Co., Ltd.) according to the instructions attached to the kit. Avidin and streptavidin are commercially available from, for example, Sigma. Tamavidin 2 is commercially available from Wako Pure Chemical Industries, Ltd.

In the use of hydrophobic bonding, binding is achieved by interaction between the hydrophobic surface of the carrier and the hydrophobic moiety of the biotin-binding protein. Specifically, a solution of the biotin-binding protein is put into direct contact with the surface of a carrier such as a microplate (e.g., but not limited to, Nunc-Immuno (trademark) Plate (Nunc), SpectraPlate-96 HB (Perkin Elmer), or Reacti-Bind (trademark) 96-Well Plates Corner Notch (PIERCE)), and is allowed to stand for a predetermined time, so that the biotin-binding protein is bound and immobilized to the carrier by the interaction between the hydrophobic moiety of the biotin-binding protein and the hydrophobic portion of the carrier.

In the covalent bonding, functional groups are provided on the surface of a carrier so as to be bound to the functional groups in the biotin-binding protein. For such binding, a variety of carriers provided with various functional groups on the surfaces is commercially available and can be preferably used. Nonlimiting examples of such microplates provided with functional groups on the surface include maleic anhydride plates, e.g., Reacti-Bind (trademark) Maleic Anhydride Activated Polystyrene 96-Well Plates (PIERCE); activated amino group plates, e.g., Immobilizer (trademark)-Amino Modules/Plates (Nunc); and carboxyl group plates, e.g., ELISA plate MS-8796F (96 wells, type C, flat bottom, Carbo) (Sumitomo Bakelite Co., Ltd). Nonlimiting examples of microbeads provided with functional groups on the surfaces include highly cross-linked agarose beads, e.g., Sepharose (trademark) (GE Healthcare Biosciences); and magnetic beads, e.g., Dynabeads (trademark) (Dynal). Linking between the biotin-binding protein and the solid carrier may be performed according to the instructions attached to the carrier.

In a nonlimiting specific embodiment, a protein can be bound to a solid carrier by a coupling process known to persons skilled in the art as follows. For example, a biotin-binding protein can be linked to a solid carrier by a coupling reaction of carboxyl groups exposed on the surface of the solid carrier by modification and amino groups of the biotin-binding protein in the presence of a cross-linking agent, 1-ethyl-3-(3-dimethylaminopropyl) carbodiimide (EDC). Alternatively, a biotin-binding protein is mixed with a solid carrier having a surface active esterified with N-hydroxysuccinimide (NHS) in a buffer solution not containing a primary amino group and having a pH of 6.5 to 9 to bind the carboxyl groups on the solid carrier surface and the amino groups of the biotin-binding protein. Alternatively, amino groups of a biotin-binding protein can be bound to amino groups of a solid carrier surface using a cross-linking agent, bis(sulfosuccinimidyl)suberate (BS3) or disuccinimidyl suberate (DSS), or thiol groups of a biotin-binding protein can be bound to amino groups of a solid carrier surface using a cross-linking agent, N-succinimidyl 3-(2-pyridyldithio)propionate (SPDP) or N-(4-maleimidobutyryloxy)succinimide (GMBS).

Examples of a carrier on which a biotin-binding protein is immobilized include, but not limited to, commercially available products: microplates such as Reacti-Bind (trademark) Streptavidin Coated Plates (PIERCE) and Nunc Streptavidin Coated 96 Micro Well (trademark) Plates (Nalge Nunc); and magnetic beads such as Dynabeads M-280 Streptavidin (Dynal) and MagnaBind (trademark) Streptavidin Beads (PIERCE).

The substance capable of specifically binding to a substance to be detected may be biotinylated by any method. For example, biotin may be bound to a specific binding substance using a biotin labeling kit (e.g., but not limited to, EZ-Link (registered trademark) NHS-Lc-Biotin (PIERCE) or Biotin Labeling Kit-NH2 (DOJINDO MOLECULAR TECHNOLOGIES INC.)). Alternatively, when the specific binding substance is a protein, a biotinylated specific binding substance may be produced by fusing a gene encoding the protein with a DNA encoding a peptide comprising a biotinylated sequence, constructing a vector expressing the fusion gene, and expressing the biotinylated specific binding protein as a fused protein with a biotinylated sequence in any host (Schwarz et al., (1988). J. Biol. Chem., 263: 9640-9645).

Nonlimiting examples of such vectors include vectors comprising BioEase (trademark) tags available from Invitrogen. Among them, a pcDNA (trademark) 6 vector is used for mammalian cell expression, a pET 104 vector for E. coli expression, and a pMT/BioEase vector for Drosophila expression.

Furthermore, preferably, a biotinylation agent can be used. Examples of the biotinylation agent include, but not limited to, EZ-Link (registered trademark) Sulfo-NHS-Biotin (the length of the linker: 13.5 angstroms, the reactive group: primary amine, hereinafter the same order), EZ-Link (registered trademark) Sulfo-NHS-LC-Biotin (22.4 angstroms, primary amine), EZ-Link (registered trademark) Sulfo-NHS-LCLC-Biotin (30.5 angstroms, primary amine), EZ-Link (registered trademark) PFP-Biotin (9.6 angstroms, amine), EZ-Link (registered trademark) Maleimide-PEO₂-Bioti (29.1 angstroms, thiol group), EZ-Link (registered trademark) Biotin-PEO₂ Amine (20.4 angstroms, carboxyl group), EZ-Link (registered trademark) Biotin-PEO₃-LC Amine (22.9 angstroms, carboxyl group), EZ-Link (registered trademark) Biotin-Hydrazide (15.7 angstroms, aldehyde group), EZ-Link (registered trademark) Biotin-LC-Hydrazide (24.7 angstroms, aldehyde group), and EZ-Link (registered trademark) NHS-Iminobiotin (13.5 angstroms, primary amine), which are available from PIERCE.

Using such a biotinylation agent, biotin can be bound to a specific binding substance through any known process. For example, in the use of a biotinylation agent containing NHS ester, biotin can be bound to a specific binding substance by dissolving biotin in an organic solvent such as a dimethyl sulfoxide (DMSO) or phosphate buffer solution (pH: 7 to 9) and adding the solution to the specific binding substance. Alternatively, in the use of a biotinylation agent containing amino groups, biotin may be bound to a specific binding substance by converting the carboxyl group of the specific binding substance into activated ester with carbodiimide such as 1-ethyl-3-(3-dimethylaminopropyl)carbodiimide hydroxychloride (EDC) and then adding a biotinylation agent dissolved in a buffer solution (pH: about 5) to the specific binding substance.

In the production of a biotinylated specific binding substance using such a biotinylation agent, the specific binding substance is preferably purified in advance.

The biotinylated specific binding substance prepared as in above is put into contact with a biotin-binding protein bound to a carrier and thereby can be immobilized to the carrier through binding between biotin and the biotin-binding protein.

### B) Process of binding a biotin-binding protein to biotin immobilized to a carrier and then binding a biotinylated substance capable of specifically binding to a substance to be detected thereto

Utilizing the fact that many of the biotin-binding protein molecules are in a tetramer form, a biotin-binding protein is immobilized to a biotinylated carrier through binding between the biotin-binding protein and biotin, and then a biotinylated specific binding substance is bound to another biotin pocket of the biotin-binding protein to immobilize biotin, the biotin-binding protein, biotin, and the specific binding substance in this order to the carrier.

The carrier can be biotinylated by the same method as biotinylation of the specific binding substance, for example, using a biotinylation agent. Examples of the biotinylation agent include those mentioned in process A), but are not limited thereto.

Using these biotinylation agent, biotin can be bound to a desired carrier such a microplate, microbeads, or a sensor chip by any known process. For example, various carriers having functional groups, such as amino, carboxyl, thiol, tosyl, epoxy, and maleimide groups and activated ester (for example, magnetic beads, Sepharose beads, agarose beads, latex beads, and microtiter plates) can be used. For example, in the use of a biotinylation agent containing NHS ester, biotin can be bound to an immobilization carrier having amino groups by dissolving biotin in an organic solvent such as a dimethyl sulfoxide (DMSO) or phosphate buffer solution (pH: 7 to 9) and adding the solution to the immobilization carrier. In the use of a biotinylation agent containing amino groups, biotin may be bound to an immobilization carrier by converting the carboxyl groups of the immobilization carrier into activated ester with carbodiimide such as 1-ethyl-3-(3-dimethylaminopropyl)carbodiimide hydroxychloride (EDC) and then adding a biotinylation agent dissolved in a buffer solution (pH: about 5) to the immobilization carrier. The biotinylated immobilization carrier is preferably blocked with BSA after inactivation of unreacted functional groups.

Commercially available biotinylated carriers can also be used. Examples of the biotinylated microplates include, but not limited to, Reacti-Bind (trademark) Biotin Coated Polystyrene Plates (PIERCE). Examples of the biotinylated microbeads include, but not limited to, magnetic beads, such as BioMag Biotin (available from Polysciences), magnetic nanobeads, such as nanomag (registered trademark)-D biotin and nanomag (registered trademark)-silica biotin available from Corefront, polystyrene microbeads, such as Beadlyte (registered trademark) Biotin Beads (available from Upstate), agarose, such as Biotin Agarose and 2-iminobiotin-Agarose available from Sigma, and highly cross-linked agarose, such as Biotin-Sepharose (available from Biosearch Technologies, Inc.).

The linker binding the carrier to biotin may have any length, which is preferably at least 5 angstroms, more preferably at least 13.5 angstroms, and most preferably at least 22.4 angstroms.

A specific binding substance can be immobilized to a carrier by bringing a biotin-binding protein into contact with a carrier biotinylated as described above to bind the biotin-binding protein to the carrier and further bringing a biotinylated specific binding substance into contact with the carrier.

### C) Process of binding a fusion protein composed of a biotin-binding protein and a substance capable of specifically binding to a substance to be detected to biotin immobilized to a carrier

In the present invention, a fusion protein of a specific binding substance (protein) and a biotin-binding protein (hereinafter may be referred to as "biotin-binding protein fusion protein" or "fusion protein") is immobilized to a biotinylated carrier.

The biotinylated carrier can be prepared as in embodiment B) described above.

The biotin-binding protein fusion protein may be prepared by any method, for example, by a known genetic engineering technique. For example, the fusion protein can be obtained by expressing a gene encoding a fusion protein of a biotin-binding protein and a desired protein using an expression system such as E. coli. Note that tamavidin and its mutants are preferred to achieve high expression in E. coli.

In the biotin-binding protein fusion protein, a biotin-binding protein may be bound to a desired specific binding substance directly or via a linker, and are preferably bound via an amino acid linker. The linker may have a length of at least one amino acid, and preferably at least five amino acids and more preferably at least six amino acids. In order to further enhance the binding strength between biotin immobilized to a carrier and tamavidin, the linker preferably has a length of at least ten amino acids, more preferably at least 12 amino acids, at least 15 amino acids, at least 18 amino acids, and most preferably at least 25 amino acids. Probably, such linkers also increase the activity of the tamavidin fusion protein. The linker may be composed of any amino acids, and the linker preferably consists of repeated neutral amino acids such as glycine, serine, or alanine. Nonlimiting examples thereof include GGGGS, GGSGG, GASAG, GSGAA, GSGSA, GGGGSG, GGGSGGS, GGSGGGGS, AAAAGSGAA, GGGGSGGGGSGGGGS, and GGGGSGGGGSGGGGSGGGGSGGGGS (SEQ ID NOs: 15 to 25).

The biotin-binding protein may be bound to either the N-terminal or the C-terminal of a desired protein. In the expression of a desired protein, for example, if the periplasmic space is more suitable than the cytoplasm of E. coli, a leader sequence for targeting the periplasm may be used. Examples of the leader sequence include, but not limited to, PelB (Lei et al., (1987), J. Bacteriol., 169: 4379-4383) and OmpA (Gentry-Weeks et al., (1992), J. Bacteriol., 174: 7729-7742).

In the case of a biotin-binding protein fusion protein obtained from a soluble fraction, the soluble fraction, without purifying the crude protein extract, may be put into contact with a biotinylated carrier to bind the fusion protein to the biotinylated carrier. Through sufficiently washing the carrier, the purification and the immobilization to the carrier of the fusion protein can be simultaneously achieved. Alternatively, the fusion protein may be bound to the biotinylated carrier after purification using a column to which a biotin analog such as iminobiotin (Hofmann et al., (1980), Proc. Natl. Acad. Sci. USA, 77: 4666-4668) is bound.

Alternatively, a tag for purification may be further added to the N-terminal or the C-terminal of the biotin-binding protein fusion protein. Examples of the tag include, but not limited to, a c-myc epitope tag (Munro and Pelham, (1986), Cell, 46: 291-300), a histidine tag (Hochuli et al., (1988), Bio/Technol, 6: 1321-1325; Smith et al., (1988), J. Biol. Chem., 263: 7211-7215), a Halo tag (Los and Wood, (2007), Methods Mol. Biol., 356: 195-208), a Flag tag (Einhauer and Jungbauer, (2001), J. Biochem. Biophys. Methods, 49: 455-465), and combinations thereof.

A fusion protein can be obtained from an insoluble fraction through a known process involving, for example, solubilization of the protein with a chaotropic salt, such as urea or guanidine hydrochloride, and then prompt of refolding of the protein by gradually removing the chaotropic salt through, for example, dialysis (Sano and Cantor, (1991), Bio/Technology, 9: 1378-1381; Sano et al., (1992), Proc. Natl. Acad. Sci. USA, 89: 1534-1538).

Alternatively, a desired protein may be coexpressed in an insoluble fraction in E. coli, for example, with a maltose-binding protein (Bach et al., (2001), J. Mol. Biol., 312: 79-93), thioredoxin (Jurado et al., (2006), J. Mol. Biol., 357: 49-61), glutathione S-transferase (Tudyka and Skerra, (1997), Protein Sci., 6: 2180-2187), or a chaperon such as one described in Ideno et al., (2004), Appl. Microbiol. Biotechnol., 64: 99-105, or a three-component fusion protein composed of the fusion protein and a chaperon may be produced. The maltose-binding protein, the thioredoxin, and the glutathione S-transferase can also be used as tags for purification.

The fusion protein may be expressed by any other known expression system, such as insect cells, plant cells, mammalian cells, yeast cells, Bacillus subtilis cells, or a cell-free expression system. In particular, when the protein to be fused is expressed in plant cells (for example, plant lectin), it is preferred that the fusion protein be also expressed in a plant cell expression system. A suitable expression system will be apparent to those skilled in the art in consideration of properties of the protein to be fused.

A biotin-binding protein fusion protein is prepared as described above and is brought into contact with a biotinylated carrier to immobilize the protein to the carrier through avidin-biotin bonding.

In each of embodiments A) to C), a substance to be detected (a biological sample containing the substance to be detected) may be bound to a substance capable of specifically binding to the substance to be detected at any time of before, during, and after the immobilization of the substance capable of specifically binding to the substance to be detected to a carrier. The monomer of a biotin-binding protein and a biological sample may also be put into contact with each other at any time.

### v) Monomer of biotin-binding protein

The present inventors have found that non-specific detection in a step of detecting a substance in a biological sample can be inhibited by bringing a monomer of a biotin-binding protein not substantially having biotin-binding ability into contact with the biological sample and have arrived at the present invention.

Accordingly, an embodiment of the method of inhibiting of the present invention includes bringing a monomer of a biotin-binding protein not substantially having biotin-binding ability into contact with a biological sample.

The monomer of a biotin-binding protein may be usually brought into contact with a biological sample at any time of before, during, and after the binding of a substance to be detected and a substance capable of specifically binding to the substance to be detected, and is preferably before or during the binding of the substance to be detected.

The details of the "monomer of a biotin-binding protein" will be described in the section "III. Monomer of biotin-binding protein".

### vi) Detection of a substance to be detected utilizing a specific binding substance-immobilized carrier

The "step detecting a substance in a biological sample" of the present invention detects the substance to be detected bound to the substance capable of specifically binding to the substance to be detected immobilized to a carrier. An embodiment of the detection step will now be described.

If the specific binding substance and/or the substance to be detected are a protein, those skilled in the art can appropriately select the step of detecting the substance to be detected based on the properties of the intended protein. Preferred examples of the step include immunoassay such as enzyme-linked immunosorbent assay (ELISA, including sandwich ELISA) and radioimmunoassay (RIA). If the specific binding substance or the substance to be detected is a nucleic acid, for example, nucleic acid hybridization assay can be employed. If the specific binding substance or the substance to be detected is a protein or a low-molecular-weight compound, for example, surface plasmon resonance can be employed. A test sample is applied to a substance specifically binding or interacting with a substance to be detected and immobilized with avidin-biotin bonding, followed by the detection of the substance to be detected. Alternatively, a test sample is first reacted with a biotinylated substance specifically binding or interacting with a substance to be detected, and the resulting complex is then put into contact with a biotin-binding protein immobilized to a carrier, followed by the detection of the substance to be detected.

In immunoassay, for example, if the substance to be measured is an antibody, an antigen is immobilized. The antibody present in a test sample is reacted with the antigen and is detected by a process known to those skilled in the art. For example, in a human-derived test sample, a human antibody bound to an antigen is detected with an anti-human antibody. In this case, the anti-human antibody is labeled with a fluorescent material, enzyme, or radioisotope; and the amount of the antibody is indirectly measured and quantified by finally measuring the fluorescent intensity, enzyme activity, or radiation dose. If the substance to be measured is an antigen, an antibody against a certain site (epitope) of the antigen is immobilized and is reacted with the antigen in a test sample; and an antibody against another epitope of the antigen is further reacted. The secondary antibody against the other epitope is labeled for indirectly measuring the amount of the antigen as in above. Alternatively, in nucleic acid hybridization assay, a nucleic acid composed of several tens of nucleotides to several hundreds or several thousands of nucleotides and having a sequence region complementary to a nucleic acid to be measured is immobilized by avidin-biotin bonding. This is reacted with a test sample containing a nucleic acid labeled in advance with a fluorescent material or radioisotope; and the fluorescent intensity or radiation dose is measured.

Any labeling well known to persons skilled in the art may be employed. Alternatively, commercially available fluorescent- or enzyme-labeled anti-human antibodies may be used. Examples of the fluorescent labeling include labeling with, for example, fluorescein or rhodamine, and labeling with a fluorescent protein such as a green fluorescent protein (GFP). Examples of the enzyme labeling include, but not limited to, labeling with peroxidase, alkaline phosphatase, luciferase, or glucose oxidase. Substrates for measurement using these enzymes are commercially available. For example, TBA and substrates for chemiluminescence can be used for peroxidase. Examples of the radioisotope include iodine (¹²⁵I and ¹²¹I), carbon (¹⁴C), sulfur (³⁵S), and tritium (³H), and phosphorus (³²P) for nucleic acids.

The amount of an antigen or an antibody present in a biological sample (specimen) can be readily calculated by comparison with the amount present in a standard preparation (e.g., a standard sample of a healthy subject or a typical patient in the case of clinical samples) using a linear regression computer algorithm. Such assay for detecting an antigen or an antibody, for example, ELISA is described in Iacobelli et al., Breast Cancer Research and Treatment, 11: 19-30 (1988).

If the specific binding substance or the substance to be detected is a nucleic acid, those skilled in the art can appropriately select the step of detecting the substance to be detected based on the properties of the intended nucleic acid. Preferred examples of the step include Southern hybridization and Northern hybridization. Alternatively, for example, but not limited to, PCR, LAMP, DNA microarray method (DNA chip method), in situ hybridization, or fluorescein in situ hybridization (FISH) can be employed.

### II. Agent for inhibiting non-specific binding in step of detecting a substance in a biological sample

The present inventors have found that non-specific binding in a step of detecting a substance in a biological sample can be inhibited by bringing a monomer of a biotin-binding protein not substantially having biotin-binding ability into contact with the biological sample and have arrived at the present invention.

An embodiment of the present invention provides an agent for inhibiting non-specific binding in a step of detecting a substance in a biological sample. The agent contains a biotin-binding protein monomer not substantially having biotin-binding ability, and the step of detecting utilizes immobilization of a substance capable of specifically binding to the substance to be detected to a carrier through binding between biotin and the biotin-binding protein before or after binding of the substance to be detected to the substance capable of specifically binding to the substance to be detected.

The present inventors have found that there are a certain number of serum samples in which non-specific binding is not prevented by adding a biotin-binding protein tetramer to the samples in the step of detecting a substance in a biological sample and have diligently studied in order to reduce non-specific binding in such serum samples. As a result, the inventors have found that a monomer of the protein can reduce the non-specific binding.

The inventors have further continued investigation and have found that such a monomer binds to a substance that binds to the "biotin-binding protein bound to biotin" (Example 5). This suggests the mechanism of inhibiting non-specific binding by a biotin-binding protein monomer, but not limited to, as follows. The binding of biotin to the biotin-binding protein causes a specific structural change in the biotin-binding protein, and thereby an epitope of the biotin-binding protein that is hidden in a usual state comes to the surface. To this epitope, some component in the sample non-specifically binds to cause a background signal. The monomer of the biotin-binding protein adsorbs such a component and thereby inhibits the non-specific binding.

The non-specific binding-inhibiting agent of the present invention includes a biotin-binding protein monomer. The biotin-binding protein from which the monomer is derived is preferably, but not limited to, the same species as that of the biotin-binding protein constituting binding between biotin and the biotin-binding protein for immobilizing the substance capable of specifically binding to the substance to be detected to a carrier. For example, in the case of immobilizing the specific binding substance to a carrier with tamavidin, the monomer of tamavidin is preferably used as the non-specific binding-inhibiting agent. In the case of immobilizing the specific binding substance to a carrier with streptavidin, the monomer of streptavidin is preferably used.

However, such a biotin-binding protein monomer is not required to have an amino acid sequence that is completely the same as that of the biotin-binding protein for immobilizing the specific binding substance to a carrier. The biotin-binding protein monomer may be a monomer constituting a mutant of the biotin-binding protein for immobilizing the specific binding substance to a carrier. For example, in the case of immobilizing a specific binding substance with native tamavidin, the monomer used as the non-specific binding-inhibiting agent is not limited to the monomer of native tamavidin and may be the monomer of a tamavidin mutant. In the present invention, the biotin-binding protein tetramer that is used as a material for producing a biotin-binding protein monomer having a non-specific binding-inhibiting function may have biotin-binding ability or may not have biotin-binding ability.

The biotin-binding protein monomer contained in the non-specific binding-inhibiting agent of the present invention may have any concentration which can be appropriately determined depending on the sample. The non-specific binding-inhibiting agent of the present invention may further contain an appropriate buffer solution or a surfactant for solubilizing the biotin-binding protein monomer, in addition to the biotin-binding protein monomer. Furthermore, the agent may contain a bacterium component used in production of the recombinant biotin-binding protein or another non-specific binding-inhibiting agent.

The biotin-binding protein monomer is not limited to being soluble and may be immobilized to a solid phase, preferably, on a film, a microtiter plate, a microcontainer, or microbeads. Non-specific binding can be inhibited by bringing a biological sample containing a substance to be detected into contact with such a solid phase on which the biotin-binding protein monomer is immobilized. Accordingly, such a form is an embodiment of the non-specific binding-inhibiting agent.

For example, a biological sample is first allowed to pass through a column to which a biotin-binding protein monomer is immobilized to adsorb non-specific binding substances in the biological sample, and the sample from the column may be then put into contact with a substance capable of specifically binding to the substance to be detected.

The details of the "monomer of a biotin-binding protein" will be described in the section "III. Monomer of biotin-binding protein".

### III. Monomer of biotin-binding protein

### i) Properties of monomer of the present invention

The present invention provides a biotin-binding protein monomer that can be used as a non-specific binding-inhibiting agent. The biotin-binding protein monomer of the present invention does not substantially have biotin-binding ability.

The biotin-binding protein monomer of the present invention preferably has, but not limited to, a molecular weight of 5 to 20 kDa. The biotin-binding protein monomer of the invention preferably does not aggregate, but is not essential.

The method of the present invention includes an embodiment involving bringing a "biotin-binding protein monomer" into contact with a biological sample and then reacting with a substance capable of specifically binding to a substance to be detected immobilized in advance to a carrier through avidin-biotin bonding. In this embodiment, if the monomer has strong biotin-binding ability, the biotin-binding protein used for immobilizing the a substance capable of specifically binding to a substance to be detected may be replaced with the monomer.

The method of present invention also includes an embodiment involving adding a biotin-binding protein monomer to a sample and reacting a biotinylated substance capable of specifically binding to a substance to be detected then with the sample and is then reacted with a biotin-binding protein-immobilized carrier. In this embodiment, if the monomer has biotin-binding activity, such a monomer increases a risk of a reaction of the monomer with the biotinylated substance capable of specifically binding to a substance to be detected to prevent the biotinylated substance capable of specifically binding to a substance to be detected from reacting with the biotin-binding protein-immobilized carrier.

Accordingly, the monomer of the present invention preferably has low biotin-binding ability and more preferably does not substantially have biotin-binding ability. Monomerization by introducing mutation into the amino acid sequence of a native biotin-binding protein constituting a tetramer has been known prior to the present invention. However, such a monomer protein has been produced for the use in technical fields that require reversible binding, such as affinity chromatography, and the biotin-binding ability required is about 10⁻⁹ to 10⁻⁶ (M). The "substantially not having biotin-binding ability" in the present invention is lower than the levels of such monomer proteins. The dissociation constant (KD) with biotin is preferably, but not limited to, 10⁻⁵ M or more, preferably 10⁻⁴ M or more, more preferably 10⁻³ M or more, and most preferably 10⁻² M or more.

The biotin-binding protein monomer of the present invention may have any molecular weight, which is preferably, but not limited to, 25 kDa or less, preferably 5 to 20 kDa, and more preferably 10 to 20 kDa.

The biotin-binding protein monomer of the present invention is preferably non-aggregatable in the absence of surfactants or under refrigeration conditions (2°C to 10°C). The term "aggregation" refers to a phenomenon of gathering a large number of denatured protein molecules or its structure.

The biotin-binding protein monomer of the present invention preferably has all properties described above. That is, the biotin-binding protein monomer according to an embodiment of the invention has the following properties i) to iii):
i) not substantially having biotin-binding ability;
ii) having a molecular weight of 5 to 20 kDa; and
iii) not aggregating.

The biotin-binding protein monomer of the present invention preferably does not reassemble into a tetramer. That is, the monomer is preferably formed irreversibly. The term "assembling" refers to binding of protein molecules (e.g., assembling of subunits).

The monomer according to an embodiment of the invention is a monomer of a biotin-binding protein, such as avidin, streptavidin, tamavidin 1, or tamavidin 2, or its mutant and has low biotin-binding ability and a property of not forming a tetramer by reassembling and is non-aggregatable under refrigeration conditions (2°C to 10°C) or room temperature (20°C to 30°C).

The monomer of the present invention may be produced by any method. For example, the monomer can be produced by treating a biotin-binding protein constituting a tetramer described above with heat or a chemical. Alternatively, the monomer may be produced by monomerization of a native biotin-binding protein constituting a tetramer by modifying the amino acid sequence of the protein.

The monomer according to an embodiment of the present invention comprises an amino acid sequence selected from the group consisting of:
i) the amino acid sequence of SEQ ID NO: 2;
ii) the amino acid sequence of SEQ ID NO: 4;
iii) the amino acid sequence of SEQ ID NO: 6;
iv) the amino acid sequence of SEQ ID NO: 8;
v) amino acid sequences having deletion, substitution, insertion, and/or addition of one or several amino acids in the amino acid sequence of SEQ ID NO: 2, 4, 6, or 8;
vi) amino acid sequences having at least 80% identity with the amino acid sequence of SEQ ID NO: 2, 4, 6, or 8;
vii) amino acid sequences encoded by nucleic acids consisting of the nucleotide sequence of SEQ ID NO: 1, 3, 5, or 7;
viii) amino acid sequences encoded by nucleic acids consisting of the nucleotide sequence having deletion, substitution, insertion, and/or addition of one or more nucleotides in the nucleotide sequence of SEQ ID NO: 1, 3, 5, or 7;
ix) amino acid sequences encoded by nucleic acids consisting of the nucleotide sequence having at least 80% identity with the nucleotide sequence of SEQ ID NO: 1, 3, 5, or 7; and
x) amino acid sequences encoded by nucleic acids hybridizable under stringent hybridization conditions with nucleic acids consisting of nucleotide sequences complementary to the nucleotide sequence of SEQ ID NO: 1, 3, 5, or 7.

The meanings of "deletion, substitution, insertion, and/or addition of one or more amino acids", "deletion, substitution, insertion, and/or addition of one or more nucleotides", "percent identity", and "stringent hybridization conditions" are the same as those described for tamavidin.

The biotin-binding protein constituting a dimer or tetramer as a raw material for preparing a monomer of the present invention is not limited, and various biotin-binding proteins and mutants thereof described in the section "I ii) biotin-binding protein" of the present specification can be suitably used. The protein described in the section "I ii) biotin-binding protein" preferably has biotin-binding ability, but the biotin-binding protein constituting a dimer or tetramer as a raw material for preparing a monomer of the present invention does not necessarily has biotin-binding ability. For example, a protein lacking biotin-binding ability prepared by mutating an amino acid of a native biotin-binding protein can also be used as a raw material for producing a monomer of the present invention.

### ii) Production of monomer by heat treatment

In the present invention, the monomer produced by heat treatment can be preferably used. The present inventors have surprisingly found that even if tamavidin or streptavidin is treated at a high temperature of 99.9°C or more, not all of the tamavidin or streptavidin molecules aggregate to generate a monomer. The resulting monomer lacks biotin-binding ability and does not form a tetramer by reassembling even after one to three months.

That is, the monomer of the present invention can be prepared by heat-treating a biotin-binding protein constituting a dimer or tetramer. The biotin-binding protein dimer or tetramer as a raw material to be heat-treated may be a native type or a mutant. The mutant may have any quaternary structure and may or may not have biotin-binding ability and the ability may be high or not, as long as the native biotin-binding protein from which the mutant is derived is a biotin-binding protein constituting a dimer or tetramer.

In the case of using tamavidin or streptavidin as the biotin-binding protein for generating a monomer, for example, the monomer can be obtained from a soluble fraction prepared by heat treating a solution of 0.05 to 0.1 mg/mL of the biotin-binding protein at 120°C for 15 min. In the case of using the resulting soluble fraction as a non-specific binding-inhibiting agent, it is important that the fraction do not contain tetramers, whereas the presence of a small amount of dimers does not interfere with the use of the fraction.

The inventors also found thermostable tamavidin N115C (SEQ ID NO: 10), which is prepared by replacing the asparagine residue at position 115 of the amino acid sequence (SEQ ID NO: 2) of native tamavidin 2 with cysteine, can maintain the biotin-binding activity even after heating at 99.9°C for 32 min (WO2012/091110). Such thermostable tamavidin can be heat-treated at a temperature higher than those for treating native tamavidin or its mutants.

In order to perform heat treatment at 100°C or more, it is necessary to adequately control the pressure. Accordingly, for example, an autoclave can be preferably used. An unpurified biotin-binding protein may be roughly purified in advance. For example, tamavidin 2 expressed in E. coli may be roughly purified by heat treatment at 80°C and then be subjected to the heat treatment described above.

### ii) Production of monomer by chemical treatment

In the present invention, a monomer can also be produced by chemical treatment.

### iii) Production of monomer by modification of amino acid sequence

The present invention can also use a monomer (variant) monomerized by modifying the amino acid sequence of a native biotin-binding protein constituting a tetramer.

For example, in avidin, streptavidin, and tamavidin, the amino acid involved in the interaction between subunits of each protein is known, and a monomer protein showing low affinity to biotin has been produced by mutating this site (Laitinen et al., (2001), J. Biol. Chem., 276: 8219-8224; Wu and Wong, (2005), J. Biol. Chem., 280: 23225-23231; PCT/JP2009/061530). In addition, for example, in streptavidin, monomer streptavidin showing low affinity to biotin has been produced by mutating the amino acid involved in the interaction with biotin (for example, Qureshi et al., (2001), J. Biol. Chem., 276: 46422-46428).

However, these monomer proteins have been produced for the use in technical fields that require reversible binding, such as affinity chromatography, and the biotin-binding ability required is about 10⁻⁹ to 10⁻⁶ (M) (for example, Qureshi et al., (2001), J. Biol. Chem., 276: 46422-46428).

In contrast, the monomer of the present invention preferably has low biotin-binding ability. As described above, the binding constant is 10⁻⁵ M or more, preferably 10⁻⁴ M or more, more preferably 10⁻³ M or more, and more preferably 10⁻² M or more; and most preferably, the monomer does not bind to biotin.

Such a monomer can be produced by mutating a plurality of amino acid residues of amino acids involved in assembling of subunits (e.g., Laitinen et al., (2001), J. Biol. Chem., 276: 8219-8224; Wu and Wong, (2005), J. Biol. Chem., 280: 23225-23231; PCT/JP2009/061530) and/or amino acids involved in interaction with biotin (e.g., Livnah et al., (1993), Proc. Natl. Acad. Sci. USA, 90: 5076-5080; Qureshi et al., (2001), J. Biol. Chem., 276: 46422-46428; Takakura et al,. (2009), FEBS J., 276: 1383-1397).

Those skilled in the art can readily produce monomer proteins of the variants described above based on the disclosure of the present specification. For example, the mutant and variant can be produced by a known method for mutating the amino acid sequence of a protein.

The biotin-binding ability of a monomer (including mutants and variants) can be examined by, for example, a method described in the section " ii) Biotin-binding protein" in "Carrier to which substance capable of specifically binding to a substance to be detected is bound through bond between biotin and biotin-binding protein". Furthermore, the molecular weight and aggregability of the monomer can also be confirmed based on the description of the present specification and/or well-known art.

The present invention also relates to a biotin-binding protein monomer that is produced by heat treatment, chemical treatment, or modification of the amino acid sequence as described above and does not substantially have biotin-binding ability. The biotin-binding protein monomer is preferably, but not limited, produced by heat treatment. The temperature of the heat treatment is, but not limited to, 90°C or more and 140°C or less, preferably 100°C or more and 130°C or less, more preferably 110°C or more and 130°C or less, and most preferably 120°C. The time of the heat treatment is, but not limited to, preferably 1 min or more and 120 min or less and can be appropriately selected by those skilled in the art depending on the selected treatment temperature. The treatment time can be decreased as the heat treating temperature is increased, but is not essential.

The concentration of the biotin-binding protein to be heated is not limited, but is 0.01 to 5 mg/mL and preferably 0.01 to 1 mg/mL. The appropriate concentration varies depending on the conditions such as the dilution solution used. Those skilled in the art can appropriately employ a suitable concentration based on the disclosure of the present specification.

An embodiment includes a biotin-binding protein monomer prepared by heat-treating tamavidin or streptavidin at 120°C for 15 min. Alternatively, a biotin-binding protein monomer may be prepared by heat-treating a biotin-binding protein having replacement of the asparagine residue at position 115 of the amino acid sequence (SEQ ID NO: 2) of native tamavidin 2 with cysteine at 120°C for 15 min.

The present invention also provides use of a biotin-binding protein monomer not substantially having biotin-binding ability of the present invention for inhibiting non-specific binding in the step of detecting a target material in a biological sample.

### IV. Kit

The present invention also provides a kit to be used for a method of inhibiting non-specific binding in a step of detecting a substance to be detected in a biological sample. The kit comprises a non-specific binding-inhibiting agent containing a biotin-binding protein monomer not substantially having biotin-binding ability of the present invention.

The kit according to an embodiment of the present invention comprises:
a substance capable of specifically binding to a substance to be detected immobilized to a carrier through a biotin-binding protein; and
a non-specific binding-inhibiting agent containing a biotin-binding protein monomer.

Alternatively, the kit according to another embodiment of the present invention comprises:
a biotinylated substance capable of specifically binding to a substance to be detected;
a biotin-binding protein immobilized to a carrier; and
a non-specific binding-inhibiting agent containing a biotin-binding protein monomer.

Alternatively, the kit according to another embodiment of the present invention comprises:
a fusion protein of a biotin-binding protein and a substance capable of specifically binding to a substance to be detected;
a biotinylated carrier; and
a non-specific binding-inhibiting agent containing a biotin-binding protein monomer.

### EXAMPLES

The present invention will now be described in detail by way of the following examples, which are not intended to limit the invention. Based on the descriptions of the specification, modifications and changes will be apparent to those skilled in the art, and such modifications and changes fall within the technical scope of the invention.

### Example 1: Preparation of biotin-binding protein monomer

Monomers of tamavidin 2 (TM2) and its mutant, TM2 N115C, were prepared. TM2 N115C is a mutant tamavidin developed by the present inventors having replacement of asparagine (N115) at position 115 of tamavidin 2 with cysteine (Cys) and is thermostable tamavidin substantially completely maintaining the biotin-binding activity even after treatment at 99.9°C for 30 min (WO2012/0911 of which in its entire is incorporated herein by reference).

A single colony of E. coli BL21 transformed with an E. coli expression vector (pTrc99A) containing a gene of tamavidin 2 (TM2) or TM2 N115C was inoculated into an Overnight Express Instant LB Medium (manufactured by Merk) containing an antibiotic, ampicillin, (final concentration: 100 µg/mL) and cultured with shaking at 37°C overnight. The cells were collected by centrifugation and were suspended in 0.1 M HEPES/KOH (pH 7.4), followed by ultrasonic homogenization. The homogenate was centrifuged, and the supernatant was diluted such that the total soluble protein concentration was 10 mg/mL measured by a Bradford method. On this occasion, the concentrations of TM2 and TM2-N115C were each about 2.5 mg/mL, and 300 mL of the culture solution provided 25 mg of protein in the case of TM2 and 28 mg of protein in the case of TM2 N115C.

### i) Rough purification of biotin-binding protein by heat treatment at 80°C

The total soluble protein fraction (10 mL) containing 10 mg/mL soluble proteins, in which the concentration of TM2 or TM2-N115C was 1 to 2.5 mg/mL, was heated at 80°C for 30 min. Subsequently, the fraction was centrifuged at 15000 rpm for 10 min. The soluble proteins in the supernatant were suspended in an equal amount of a 2 × SDS sample buffer (100 mM Tris-HCl, pH 6.8, 200 mM DTT, 2% SDS, 20% glycerol) and were heated at 99°C for 10 min and were then subjected to SDS-PAGE. The protein bands were detected by CBB staining and were quantitatively measured with Las-3000 (FUJIFILM) using a calibration curve formed based on quantitative markers (LMW ELECTROPHORESIS CALIBRATION KIT; Pharmacia Biotech).

The results demonstrated that the concentrations of TM2 and TM2-N115C contained in the 80°C heat treatment fraction were each about 1.6 mg/mL.

### ii) Investigation of monomerization of biotin-binding protein by heat treatment

The total soluble protein fraction of E. coli expressing TM2-N115C, a fraction roughly purified by heat treatment of the total soluble protein fraction at 80°C, and a TM2-N 115 C fraction purified through an iminobiotin affinity column were each diluted to give concentrations of 1.6, 0.8, 0.5, 0.25, 0.1, 0.05, 0.025, and 0.01 mg/mL of TM2-N1 15C protein (20 mM Kpi, pH 7.5) and were heat-treated at 120°C for 15 min. Each solution was then centrifuged at 15000 rpm for 10 min, and the supernatant was collected. The collected solution was subjected to SDS-PAGE by the method described above to measure the amount of biotin-binding protein contained in the solution from each band.

As a result, monomer fractions substantially not containing tetramers were obtained from the total soluble protein fraction of E. coli and the fraction roughly purified by 80°C heat treatment in the concentration range of 0.025 to 0.25 mg/mL of the biotin-binding protein. In the fraction purified through the affinity column, monomer fractions substantially not containing tetramers were provided in the concentration range of 0.01 to 0.5 mg/mL of the biotin-binding protein. In all of the total soluble protein fraction of E. coli, the fraction roughly purified by 80°C heat treatment, and the fraction purified through the affinity column, the amount of the biotin-binding protein in the supernatant was the highest when each fraction was heat-treated at 120°C for 15 min in the concentration range of 0.05 to 0.1 mg/mL of the biotin-binding protein. In the total soluble protein fraction of E. coli, a small amount of E. coli-derived proteins other than the biotin-binding protein was contained when the fraction was directly heat-treated at 120°C.

The temperature and the time of heating were investigated in detail using TM2 and TM2-N115C. Specifically, a solution of 0.1 mg/mL of each biotin-binding protein of the fractions of TM2 and TM2-N115C roughly purified by 80°C heat treatment was heat-treated at 90, 99.9, 105, 110, 120, or 130°C for 0, 5, 10, 15, 30, or 60 min and was then centrifuged at 15000 rpm for 10 min. Each supernatant was collected, and the collected solution was mixed with a SDS sample buffer (not containing DTT). The mixture was subjected to SDS-PAGE without heat treatment, followed by staining with CBB.

### iii) Preparation of biotin-binding protein monomer by heat treatment

A solution of 0.1 mg/mL biotin-binding protein of a fraction prepared by roughly purifying the total soluble protein fraction of E. coli expressing TM2 or TM2-N115C by heat treatment at 80°C (roughly purified fraction by 80°C heat treatment) was heat-treated at 120°C for 15 min, followed by cooling to room temperature. The solution was centrifuged at 15000 rpm for 10 min, and the soluble proteins in the supernatant were collected. Separately, a purified streptavidin product (Sigma) (concentration: 0.1 mg/mL) was heat-treated at 120°C for 15 min, followed by cooling to room temperature. The cooled solution was centrifuged, and the soluble proteins in the supernatant were collected. The collected heat-treated soluble fractions were each mixed with an SDS sample buffer (DTT+), and the mixture was treated at 99°C for 10 min, followed by SDS-PAGE, staining with CBB, and quantitative measurement with a densitometer (Fig. 1). The results demonstrated that the amount of soluble proteins after heating at 120°C was about 70% that before the heating in both TM2 and TM2-N115C, whereas the amount in streptavidin was about 100%.

The collected heat-treated soluble fraction was mixed with an SDS sample buffer (not containing DTT). The mixture was subjected to SDS-PAGE without heat treatment, followed by staining with CBB. Since no reducing agent was used and no heat treatment was performed under this condition, the biotin-binding protein forming a tetramer is detected as a tetramer. As shown in Fig. 2, no tetramers of TM2, TM2-N115C, and streptavidin were detected in the groups of heat treatment at 120°C, and it was demonstrated that these biotin-binding proteins were substantially completely monomerized.

### Example 2: Biotin-binding activity of biotin-binding protein monomer prepared by heat treatment

### Biotinylated HRP binding test

The biotin-binding activity of the biotin-binding protein monomerized by heat treatment at 120°C in iii) of Example 1 was measured by a biotinylated HRP binding test. The soluble fraction before and after the heat treatment at 120°C was mixed with an SDS sample buffer (DTT-) and was subjected to SDS-PAGE (without heat treatment), followed by blotting to a PVDF membrane (Immobilon, Millipore). The membrane after the blotting was blocked with 3% BSA/TBS overnight and was then reacted with Biotinylated HRP (VECTOR). After washing, the HRP was detected with ECL Detection Reagents (GE Healthcare).

The results showed that the biotin-binding protein substantially completely lost the biotin-binding activity by heat treatment at 120°C (Fig. 3).

### Example 3: Mass preparation of biotin-binding protein monomer by heat treatment

Subsequent to Example 2, a large amount of each monomerized biotin-binding protein was prepared by heat-treating, at 120°C for 15 min, 800 mL of a solution of 0.1 mg/mL the biotin-binding protein of each of the fractions roughly purified by 80°C heat treatment of TM2 and TM2-N115C, followed by cooling to room temperature. The denatured proteins contained in the solution were removed by filtration through a filter of 0.45 µm pore size. The soluble proteins were concentrated by mixing the filtrate with 70% saturated ammonium sulfate at 4°C overnight. The solution containing ammonium sulfate was centrifuged at 14500 rpm (25000 g) for 10 min. The resulting precipitate was dissolved in 25 mL of 20 mM Kpi (pH 7.5), followed by dialysis against 20 mM Kpi (pH 7.5). The dialyzed solution was centrifuged at 15000 rpm for 10 min. The soluble proteins in the supernatant were collected and were filtered through a filter of 0.45 µm pore size again for completely removing the precipitate, followed by addition of sodium azide at a concentration of 0.01%.

The resulting samples were subjected to the following experiments.

### Example 4: Effect of inhibiting serum non-specific binding by inactivated biotin-binding protein monomer

### i) Production of biotin-immobilized magnetic beads

To Dynabeads M-270 Amine (manufactured by Dynal), 10 mM NHS-PEG12-Biotin (PIERCE) was added. Amino groups and NHS active ester groups were reacted with each other by end-over-end mixing at room temperature for 30 min to form covalent bonds. The magnetic beads were then washed with a 0.1% BSA/0.01% Tween 20/PBS solution four times. The resulting magnetic beads were used as biotinylated magnetic beads.

### ii) Production of TM2-immobilized magnetic beads

The biotinylated magnetic beads prepared in i) and a solution of 1 mg/mL TM2 were mixed at a volume ratio of 1:1 by end-over-end mixing at room temperature for 1 hr for immobilizing TM2 through binding between TM2 and biotin. The beads were then washed with a 0.2% Tween 10/TBS solution four times and were suspended in 0.01% sodium azide/PBS of the same volume as that of the biotinylated magnetic beads.

As a result, TM2-immobilized magnetic beads in which TM2 was immobilized to the biotinylated magnetic beads were prepared.

### iii) ELISA analysis

The TM2-immobilized magnetic beads (5 µL) prepared in ii) were added to 200 µL of a solution of a human serum (Kohjin Bio, #R168334) showing non-specific binding to the TM2-immobilized magnetic beads and diluted into 400-fold with 2% BSA/PBS or 2% BSA/E. coli (BL21 cell line) crude extract (5 mg/mL of total soluble protein)/HEPES (pH 7.4), followed by mixing at room temperature for 1 hr to react the serum with TM2 immobilized to the magnetic beads.

On this operation, in order to inhibit non-specific binding, the monomer of the biotin-binding protein, TM2 or TM2-N115C, prepared in large amounts by heat treatment in Example 3 or TM2 (tetramer) purified utilizing iminobiotin affinity was added to the reaction solution at 0, 0.1, 0.2, or 0.4 mg/mL. The magnetic beads were collected with a magnet (DynaMag-96 Side, Veritas) and were washed with a 0.2% Tween 20/TBS solution three times. Human IgG non-specifically bound to TM2 immobilized to the beads was detected as follows: 200 µL of a solution of horseradish peroxidase-labeled goat anti-human IgG antibody diluted into 5000-fold with 2% BSA/PBS was mixed with the magnetic beads for 1 hr at room temperature. The beads were then washed with a 0.2% Tween 20/TBS solution three times, and 100 µL of 1 step ELISA Ultra TMB (PIERCE) was added thereto. After the system was left at room temperature for 2 min, the reaction was terminated with 100 µL of 2 M sulfuric acid, and the degree of coloring was measured with a plate reader, Infinite M200 (TECAN). This experiment was repeated twice, and the average and standard deviation were calculated.

The addition of native TM2 (affinity purification) forming a tetramer slightly inhibited the non-specific binding of human IgG to the TM2-immobilized magnetic beads, but the effect was not significant. On the contrary, the non-specific binding of human IgG to the TM2-immobilized magnetic beads was significantly inhibited by the addition of the TM2 monomer or the TM2-N115C monomer (Fig. 4). The results thus demonstrate that the TM2 monomer and the TM2-N115C monomer can significantly inhibit non-specific binding of human IgG, which could not be inhibited by the addition of the TM2 tetramer.

The monomer fractions of TM2 and TM2-N115C used in the test were hardly decomposed and maintained the monomer states even after storage at 4°C for one month and substantially completely maintained the effect of inhibiting non-specific binding. Thus, high storage stability was also proved.

### Example 5: Investigation of mechanism of effect of inhibiting serum non-specific binding by inactivated biotin-binding protein monomer

### i) Mass preparation of biotin-binding protein monomer by heat treatment

Causes of the effect of inhibiting non-specific binding by inactivated TM2 were investigated as follows: 800 mL of a solution of 0.1 mg/mL biotin-binding protein of each of a fraction roughly purified by 80°C heat treatment of TM2 and affinity purified TM2 was heat-treated at 120°C for 15 min, followed by cooling to room temperature. The denatured proteins contained in the solution were removed by filtration through a filter of 0.45 µm pore size. The soluble proteins were concentrated by mixing the filtrate with 70% saturated ammonium sulfate at 4°C overnight. The solution containing ammonium sulfate was centrifuged at 14500 rpm (25000 xg) for 10 min. The resulting precipitate was dissolved in 25 mL of 20 mM Kpi (pH 7.5), followed by dialysis against 20 mM Kpi (pH 7.5). The dialyzed solution was centrifuged at 15000 rpm for 10 min. The soluble proteins in the supernatant were collected and were filtered through a filter of 0.45 µm pore size again for completely removing the precipitate, followed by addition of sodium azide at a concentration of 0.01 %. The collected heat-treated soluble fractions, AC-TM2 and AC-purified TM2, were each mixed with an SDS sample buffer (DTT+). The mixture was treated at 99°C for 10 min, followed by SDS-PAGE, staining with CBB, and quantitative measurement with a densitometer. The results showed that the concentrations of the AC-TM2 and AC-purified TM2 were 0.4 mg/mL and 0.2 mg/mL, respectively. A control solution (AC-BL21) was prepared from an E. coli BL21 extract by rough purification through heat treatment at 80°C and further autoclave treatment (120°C, 15 min).

### ii) Preparation of biotin binding TM2

To 6 mL (corresponding to 6 mg) of a solution of 1 mg/mL TM2 (native, tetramer), in which 0.4 µmol of biotin pockets were present, 40 µmol of biotin corresponding to 100 times the amount of the biotin pockets was added, followed by end-over-end mixing at room temperature for 1 hr. The resulting biotin binding TM2 solution was dialyzed against 20 mM Kpi (pH 7.5), and the dialyzed solution was mixed with an SDS sample buffer (DTT+). The mixture was treated at 99°C for 10 min, followed by SDS-PAGE, staining with CBB, and quantitative measurement with a densitometer.

### iii) ELISA analysis

The TM2-immobilized magnetic beads (5 µL) prepared in ii) of Example 4 were added to 200 µL of a solution of a human serum (Kohjin Bio, #R168334 and #R168216) showing non-specific binding to the TM2-immobilized magnetic beads diluted into 400-fold with 2% BSA/PBS or 2% BSA/E. coli (BL21 cell line) crude extract (5 mg/mL of total soluble protein)/HEPES (pH 7.4), followed by mixing at room temperature for 1 hr to react the serum with TM2 immobilized to the magnetic beads.

On this operation, in order to inhibit non-specific binding, the monomer of TM2 roughly purified at 80°C (AC-TM2) and the monomer of affinity purified TM2 (AC-purified TM2), prepared in large amounts by heat treatment as described above, and biotin binding TM2 (tetramer) and autoclave-treated (120°C, 15 min) E. coli extract (AC-BL21) were added to the reaction solutions at 0, 0.1, 0.2, and 0.3 mg/mL (in AC-purified TM2, 0, 0.05, 0.1, and 0.15 mg/mL). As a control, a section using TM2-free biotinylated magnetic beads and AC-TM2 as the non-specific binding-inhibiting agent was tested.

The magnetic beads were collected with a magnet (DynaMag-96 Side, Veritas) and were washed with a 0.2% Tween 20/TBS solution three times. Human IgG non-specifically bound to TM2 immobilized to the beads was detected as follows: 200 µL of a solution of horseradish peroxidase-labeled goat anti-human IgG antibody diluted into 5000-fold with 2% BSA/PBS was mixed with the magnetic beads for 1 hr at room temperature. The beads were then washed with a 0.2% Tween 20/TBS solution three times, and 100 µL of 1 step ELISA Ultra TMB (PIERCE) was added thereto. After the system was left at room temperature for 2 min, the reaction was terminated with 100 µL of 2 M sulfuric acid, and the degree of coloring was measured with a plate reader, Infinite M200 (TECAN). This experiment was repeated twice, and the average and standard deviation were calculated.

The results are shown in Fig. 5. In the test using a serum #R168334, non-specific binding of human IgG to the TM2-free biotinylated magnetic beads hardly occurred, whereas non-specific binding to TM2-immobilized magnetic beads of human IgG was detected. This non-specific binding was barely inhibited with native TM2, but was inhibited by the addition of AC-TM2 (Fig. 5). The results demonstrate that non-specific binding of human IgG to the TM2-immobilized magnetic beads is not binding to the surfaces and biotin immobilized to the surfaces of the magnetic beads but is binding to TM2 and that this non-specific binding cannot be inhibited by the addition of native TM2 forming a tetramer.

The results suggest a possibility that the serum #R168334 contains an antibody that hardly reacts with native TM2 but reacts with TM2 immobilized to the magnetic beads. Accordingly, a section using biotin binding TM2 (tetramer) as the non-specific binding-inhibiting agent and a section using native TM2 (tetramer) as the non-specific binding-inhibiting agent were tested, which showed that biotin binding TM2 inhibited non-specific binding not inhibited by native TM2 (Fig. 6). Therefore, it was believed that the serum #R168334 contained an antibody recognizing TM2 bound to biotin. It was believed that AC-TM2 had a structure specifically generated as a result of binding of tamavidin 2 to biotin and appearing on the surface as an epitope and thereby showed the effect as an inhibiting agent.

There was no significant difference between AC-TM2 prepared by autoclave treatment (120°C, 15 min) of E. coli extract roughly purified at 80°C and AC-purified TM2 prepared by autoclave treatment (120°C, 15 min) of affinity purified TM2. This demonstrates that the inactivated TM2 monomer prepared by autoclave treatment mainly effectively inhibit non-specific binding (Fig. 7).

The effect of inhibiting non-specific binding of a serum #R168216 was high in the order of AC-TM2, biotin binding TM2 = native TM2, and AC-purified TM2 in the PBS system (the left in Fig. 8). The system of E. coli extract showed the same tendency (the right in Fig. 8). These results suggest that this serum reacts with TM2 and a small amount of E. coli components contaminating the TM2; and the obvious difference in the effect between AC-TM2 and AC-purified TM2 in the PBS system suggests that the inactivated TM2 monomer and also E. coli components contaminating the AC-TM2 have the effect of inhibiting non-specific binding.

### Example 6: Storage test ofbiotin-bindin^{g p}rotein monomer

### i) Investigation of stability of protein by storage at 4°C

TM2 and TM2-N115C (N115C) monomerized by heat treatment were each diluted to 0.4 mg/mL TM2 in 20 mM Kpi (pH 7.0), followed by storage at 4°C for one month or three months. Each solution was then mixed with the same amount of a 2 ×SDS sample buffer (100 mM Tris-HCl, pH 6.8, 200 mM DTT, 2% SDS, 20% glycerol). The mixture was heat-treated at 99°C for 10 min and was then subjected to SDS-PAGE. Separately, each solution reserved at 4°C for one month or three months was mixed with an SDS sample buffer not containing DTT, and the mixture was subjected to SDS-PAGE without performing heat treatment. The proteins were detected by CBB staining, and the molecular weights were calculated with LMW-SDS Marker Kit (GE healthcare).

The results demonstrated that the TM2 monomer and the N115C monomer each had a molecular weight of about 10 to 17 kDa. This revealed that decomposition substantially did not occur by storage for at least three months. In the test section not containing DTT and not performed heat treatment, no band was detected at the position of tetramer. This demonstrates that reassembling did not occur and the monomer form was maintained even after storage at 4°C for at least three months (Figs. 9, 10, and 11).

### ii) Investigation of aggregation in storage at 4°C

TM2 and TM2-N115C (N115C) monomerized by heat treatment were each diluted to 0.4 mg/mL TM2 in 20 mM Kpi (pH 7.0) and were reserved at 4°C for two weeks or three months. Each solution was then centrifuged at 15000 rpm for 10 min at 4°C, and the supernatant was collected. The precipitation fraction was mixed with 0.1 M HEPES/KOH (pH 7.4) containing 8 M urea in the same amount of the supernatant. The resulting supernatant and the precipitation fraction were each mixed with the same amount of a 2 × SDS sample buffer (100 mM Tris-HCl, pH 6.8, 200 mM DTT, 2% SDS, 20% glycerol). The mixture was heat-treated at 99°C for 10 min and was then subjected to SDS-PAGE. The proteins were detected by CBB staining.

The results demonstrated that the TM2 monomer and the N115 monomer were detected only in the supernatant even after storage at 4°C for two weeks or three months. This revealed that the TM2 monomer and the N115C monomer were dissolved in the buffer solution without precipitating by aggregation even after storage at 4°C for at least three months (Fig. 12).

### Reference Example: TM2 mutant TM2 N115C

### 1. Design of modified tamavidin 2 for improving thermal stability

Mutant TM2 N115C was constructed by replacing N115 of tamavidin 2 with cysteine (Cys).

### 2. Construction of mutant tamavidin 2 gene and expression in E. coli

### 2-1. Gene construction

PCR was performed using plasmid TM2/pTrc99A (WO02/072817), E. coli expression vector pTrc99A containing a tamavidin 2 gene, as the template. Table 1 shows the sequences (SEQ ID NOs: 11 to 14) of primers used.

**[Table 1]**

| | Primers for constructing TM2, N115 5C gene | |
|---|---|---|
| Name | Sequence (5'-3') | Length |
| Tm2NtemiPci | AAA *ACATG*T CAGACGTTCAATCTTC | 25mer (SEQ ID NO: 11) |
| Tm2CtermBam | TTT GGATCC *TTA*CTTCAACCTCCTGG | 28mer (SEQ ID NO: 12) |
| | | |
| TM2 N115C FW | CTTGTGGGGtgtG-ATTCGTTT | 21mer (SEQ ID NO: 13) |
| TM2 N115C RV | AAACGAATCacaCCCCACAAG | 21mer (SEQ ID NO: 14) |

The underlines indicate restriction enzyme sites. The italic bold letters indicate start and stop codons. Substituted codons are indicated with small letters.

### TM2-N115C

PCR was performed using plasmid TM2/pTrc99A containing TM2 as the template and using primers of Tm2NtermPci and TM2N115C RV or Tm2CtermBam and TM2N115C FW. The resulting products were each purified by agarose gel electrophoresis using low-melting agarose. The second PCR (overlapping PCR) was performed using the purified two PCR products as the templates and using primers Tm2NtermPci and Tm2CtermBam. The PCR was accomplished in 50 µL reaction solution containing the plasmid, 10 x PyroBest buffer (5 µL, TaKaRa), 2.5 mM dNTP (4 µL), primers (25 pmoles each), and PyroBest DNA polymerase (0.5 µL, TaKaRa) under reaction conditions: one cycle of 96°C for 3 min; 15 cycles of 96°C for 1 min, 55°C for 1 min, and 72°C for 1 min; and one cycle of 72°C for 6 min.

The PCR product was cloned into vector pCR4BluntTOPO (Invitrogen). The plasmid vector was introduced into E. coli TB1 by electroporation, and the plasmid DNA was extracted in a routine manner (Sambrook et al., 1989, Molecular Cloning, A laboratory manual, 2nd edition). The nucleotide sequence of the PCR product was determined from both ends.

Clones in which intended mutation was observed were digested with PciI and BamHI, and digestion products were purified by electrophoresis using low-melting agarose. The E. coli expression vector was prepared by digesting pTrc99A with restriction enzymes NcoI and BamHI. The DNA fragments prepared by the restriction enzyme treatment and the vector were ligated with Ligation Kit (TaKaRa). The ligation product was transformed into E. coli BL21, and clones containing the inserted gene were determined by colony PCR and were used for expression experiments.

### 2-2. E. coli expression

A single colony of E. coli containing tamavidin 2 or the tamavidin 2 mutant produced as in above was inoculated into an LB medium containing an antibiotic, ampicillin, (final concentration: 100 µg/mL) and cultured with shaking at 37°C overnight. The cells were inoculated into an LB medium containing ampicillin and cultured at 37°C for 2 hr. Isopropyl-ß-D(-)-thiogalactopyranoside (IPTG) was added to the cells at a final concentration of 1 mM to induce expression, and the cells were further cultured for 5 to 6 hr.

The cells were collected by centrifugation and were suspended in 50 mM CAPS (pH 12) containing 50 mM NaCl, followed by ultrasonic homogenization. The homogenate was centrifuged. 2-Iminobiotin agarose (SIGMA) was added to the supernatant, and the pH of the solution was adjusted to 12 with NaOH, followed by incubation at room temperature. The mixture was packed in an open column, and the column was sufficiently washed with 50 mM CAPS (pH 12) containing 500 mM NaCl in a volume 10 times that of the column, and the protein was eluted with 50 mM NH₄OAc (pH 4) in a volume in a volume 5 times that of the column. The eluate fraction was dialyzed with 0.1 M HEPES (pH 7.4) containing 50 mM NaCl and was used in the following analysis. In TM2-N115C, 300 mL of the culture solution provided about 14 mg of purified protein. The resulting TM2-N115C had a purity of 95% or more.

### Example 7: Effect of inhibiting serum non-specific binding by inactivated streptavidin monomer

### i) Production of biotin-immobilized magnetic beads

Biotin-immobilized magnetic beads were prepared as in the method in i) of Example 4.

### ii) Production of streptavidin-immobilized magnetic beads

The biotinylated magnetic beads prepared in i) and 1 mg/mL of a streptavidin solution were mixed at a volume ratio of 1:1 by end-over-end mixing at room temperature for 1 hr for immobilizing streptavidin to the magnetic beads through binding between streptavidin and biotin. The beads were then washed with a 0.2% Tween 10/TBS solution four times and were suspended in 0.01% sodium azide/PBS in the same volume as that of the biotinylated magnetic beads.

As a result, streptavidin-immobilized magnetic beads in which streptavidin was immobilized to biotinylated magnetic beads were prepared.

### iii) ELISA analysis

The streptavidin-immobilized magnetic beads (5 µL) were added to 200 µL of a solution of a human serum (Kohjin Bio, #N110295) showing non-specific binding to the streptavidin-immobilized magnetic beads diluted into 100-fold with 2% BSA/PBS or 2% BSA, followed by mixing at room temperature for 1 hr to react the serum with streptavidin immobilized to the magnetic beads. In order to inhibit non-specific binding during the process, streptavidin or the monomer of streptavidin prepared by heat treatment described in (iii) of Example 1 was added to the reaction solution into a concentration of 0 or 0.4 mg/mL. As a control for measuring non-specific binding to the magnetic beads, a section using biotinylated magnetic beads was tested.

The magnetic beads were collected with a magnet (DynaMag-96 Side, Veritas) and were washed with a 0.2% Tween 20/TBS solution three times. Human IgG non-specifically bound to streptavidin immobilized to the beads was detected as follows: 200 µL of a solution of horseradish peroxidase-labeled goat anti-human IgG antibody diluted into 10000-fold with 2% BSA/PBS was mixed with the magnetic beads for 1 hr at room temperature. The beads were then washed with a 0.2% Tween 20/TBS solution three times. After removal of the washing solution, the streptavidin-immobilized magnetic beads were suspended in 50 µL of Stable Peroxide Solution of SuperSignal ELISA Femto Maximum Sensitivity Substrate (TaKaRa Bio). The Luminol/Enhancer solution of SuperSignal ELISA Femto Maximum Sensitivity Substrate (TaKaRa Bio) was set to the injector of a plate reader TriStar LB941BvTi. The suspension of the streptavidin-immobilized magnetic beads was transferred to Optiplatate-96 (PerkinElmer) and was set to the plate reader TriStar LB941 vTi. The Luminol/Enhancer solution (50 µL) was inject from the injector to each well containing the streptavidin-immobilized magnetic beads, and the amount of luminescence was mesured for 30 min. This experiment was repeated twice, and the average and standard deviation were calculated (Table 2).

**[Table 2]**

| Serum | Test section | Accumulated luminescence intensity for 30 mn (RLU) | Average | Standard déviation | %CV |
|---|---|---|---|---|---|
| N110295 | biotin beads | 7,269,308 | 7.394,929 | 177,64 | 2.4 |
| | | 7,520,549 | | | |
| | - | 12,553,222 | 12,450,339 | 145,499 | 1.2 |
| | | 1347,455 | | | |
| | StAvi | 10,791,172 | 1,0,620,977 | 226,551 | 2.1 |
| | | 10,460,781 | | | |
| | AC-StAvi | 9,671,649 | 9,413,093 | 365,653 | 3.9 |
| | | 9,154,534 | | | |

In order to eliminate the influence of non-specific binding to the magnetic beads from measured values, the value obtained by subtracting the measured value in biotinylated magnetic beads from the measured value in streptavidin-immobilized magnetic beads was used as the amount of human IgG bound to streptavidin (Fig. 13).

As shown in Fig. 13, the addition of streptavidin forming a tetramer slightly inhibited the non-specific binding of human IgG to the streptavidin-immobilized magnetic beads, but the effect was not significant. On the contrary, the non-specific binding of human IgG to the streptavidin-immobilized magnetic beads was significantly inhibited by the addition of the streptavidin monomer (Fig. 13). The results thus demonstrate that the streptavidin monomer can inhibit non-specific binding of human IgG, which could not be inhibited by addition of the streptavidin tetramer.

### SEQUENCE LISTING FREE TEXT

SEQ ID NOs: 1 and 2 respectively denote the nucleotide sequence and the amino acid sequence of tamavidin 2.
SEQ ID NOs: 3 and 4 respectively denote the nucleotide sequence and the amino acid sequence of tamavidin 1.
SEQ ID NOs: 5 and 6 respectively denote the nucleotide sequence and the amino acid sequence of streptavidin.
SEQ ID NOs: 7 and 8 respectively denote the nucleotide sequence and the amino acid sequence of avidin.
SEQ ID NOs: 9 and 10 respectively denote the nucleotide sequence and the amino acid sequence of a tamavidin 2 mutant TM2 N115C.
SEQ ID NOs: 11 to 14 denote nucleotide sequences of primers used in PCR for preparing TM2 N115C.
SEQ ID NOs: 15 to 25 denote amino acid sequence examples of linkers that can be used for producing fusion proteins.

## Claims

1. A method of inhibiting non-specific binding in a step of detecting a substance in a biological sample, the method comprising:
bringing a biotin-binding protein monomer not substantially having biotin-binding ability into contact with the biological sample, wherein
in the step of detecting, a substance capable of specifically binding to the substance to be detected is immobilized to a carrier through binding between biotin and the biotin-binding protein before or after binding of the substance to be detected to the substance capable of specifically binding to the substance to be detected.

2. The method of inhibiting according to Claim 1, wherein the biotin-binding protein monomer has a molecular weight of 5 to 20 kDa.

3. The method of inhibiting according to Claim 1 or 2, wherein the biotin-binding protein monomer is non-aggregatable.

4. The method of inhibiting according to any one of Claims 1 to 3, wherein the biotin-binding protein monomer is non-aggregatable in the absence of surfactants or at a refrigeration temperature of 2°C to 10°C.

5. The method of inhibiting according to any one of Claims 1 to 4, wherein the biotin-binding protein monomer is prepared by treating a biotin-binding protein with heat or a chemical, or prepared by modifying one or more amino acid residues in the amino acid sequence of a biotin-binding protein.

6. The method of inhibiting according to any one of Claims 1 to 4, wherein the biotin-binding protein monomer is prepared by treating a biotin-binding protein with heat.

7. The method of inhibiting according to any one of Claims 1 to 6, wherein the biotin-binding protein is selected from the group consisting of tamavidin 2, tamavidin 1, streptavidin, and avidin.

8. The method of inhibiting according to any one of Claims 1 to 7, wherein the biotin-binding protein from which the monomer is derived is the same species as that of the biotin-binding protein constituting binding between biotin and the biotin-binding protein for immobilizing the substance capable of specifically binding to the substance to be detected to the carrier.

9. The method of inhibiting according to any one of Claims 1 to 8, wherein the substance capable of specifically binding to the substance to be detected used in the step of detecting is immobilized to the carrier through binding between biotin and the biotin-binding protein by any of the following A) to C):
A) binding a biotinylated substance capable of specifically binding to the substance to be detected to the biotin-binding protein immobilized to the carrier;
B) binding the biotin-binding protein to biotin immobilized to the carrier and then binding a biotinylated substance capable of specifically binding to the substance to be detected thereto; or
C) binding a fusion protein composed of the biotin-binding protein and the substance capable of specifically binding to the substance to be detected, to biotin immobilized to the carrier.

10. An agent for inhibiting non-specific binding in a step of detecting a substance in a biological sample, the agent comprising:
a biotin-binding protein monomer not substantially having biotin-binding ability, wherein
in the step of detecting, a substance capable of specifically binding to the substance to be detected is immobilized to a carrier through binding between biotin and a biotin-binding protein before or after binding of the substance to be detected to the substance capable of specifically binding to the substance to be detected.

11. The agent according to Claim 10, wherein the biotin-binding protein monomer has a molecular weight of 5 to 20 kDa.

12. The agent according to Claim 10 or 11, wherein the biotin-binding protein monomer is non-aggregatable.

13. The agent according to any one of Claims 10 to 12, wherein the biotin-binding protein monomer is non-aggregatable in the absence of surfactants or at a refrigeration temperature of 2°C to 10°C.

14. The agent according to any one of Claims 10 to 13, wherein the biotin-binding protein monomer is prepared by treating a biotin-binding protein with heat.

15. The agent according to any one of Claims 10 to 14, wherein the biotin-binding protein is selected from the group of consisting of tamavidin 2, tamavidin 1, streptavidin, and avidin.

16. A biotin-binding protein monomer having the following properties i) to iii):
i) not substantially having biotin-binding ability;
ii) having a molecular weight of 5 to 20 kDa; and
iii) not aggregating.

17. The monomer according to Claim 16 comprising an amino acid sequence selected from the group consisting of:
i) the amino acid sequence of SEQ ID NO: 2;
ii) the amino acid sequence of SEQ ID NO: 4;
iii) the amino acid sequence of SEQ ID NO: 6;
iv) the amino acid sequence of SEQ ID NO: 8;
v) amino acid sequences having deletion, substitution, insertion, and/or addition of one or several amino acids in the amino acid sequence of SEQ ID NO: 2, 4, 6, or 8;
vi) amino acid sequences having at least 80% identity with the amino acid sequence of SEQ ID NO: 2, 4, 6, or 8;
vii) amino acid sequences encoded by nucleic acids consisting of the nucleotide sequence of SEQ ID NO: 1, 3, 5, or 7;
viii) amino acid sequences encoded by nucleic acids consisting of the nucleotide sequence having deletion, substitution, insertion, and/or addition of one or more nucleotides in the nucleotide sequence of SEQ ID NO: 1, 3, 5, or 7;
ix) amino acid sequences encoded by nucleic acids consisting of the nucleotide sequence having at least 80% identity with the nucleotide sequence of SEQ ID NO: 1, 3, 5, or 7; and
x) amino acid sequences encoded by nucleic acids hybridizable under stringent hybridization conditions with nucleic acids consisting of nucleotide sequences complementary to the nucleotide sequence of SEQ ID NO: 1, 3, 5, or 7.

18. The biotin-binding protein monomer according to Claim 16 or 17, prepared by treating a biotin-binding protein with heat.

19. A use of a biotin-binding protein monomer not substantially having biotin-binding ability for inhibiting non-specific binding in a step of detecting a substance in a biological sample.

20. A kit for a method of inhibiting non-specific binding in a step of detecting a substance in a biological sample, the kit comprising a non-specific binding-inhibiting agent containing a biotin-binding protein monomer not substantially having biotin-binding ability.


SEQUENCE LISTING

<110>  JAPAN TOBACCO INC

<120>  Method for inhibiting non-specific detection in a process for
detecting a substance in a biological sample

<130>  FA0392-12099

<150>  JP 2011-168422
<151>  2011-08-01

<160>  25

<170>  PatentIn version 3.3

<210>  1
<211>  426
<212>  DNA
<213>  Pleurotus cornucopiae

<220>
<221>  CDS
<222>  (1)..(426)

<400>  1
atg tca gac gtt caa tct tca ctc acc gga acc tgg tac aat gaa ctc      48
Met Ser Asp Val Gln Ser Ser Leu Thr Gly Thr Trp Tyr Asn Glu Leu
1               5                   10                  15

aac tcc aag atg gaa ttg act gca aac aaa gac ggt act ctc act gga      96
Asn Ser Lys Met Glu Leu Thr Ala Asn Lys Asp Gly Thr Leu Thr Gly
            20                  25                  30

aag tac ctc tcc aaa gtt ggg gat gtc tac gtg ccc tac cca ctc tct     144
Lys Tyr Leu Ser Lys Val Gly Asp Val Tyr Val Pro Tyr Pro Leu Ser
        35                  40                  45

ggt cgc tat aac ctc caa ccc ccc gcg gga caa ggc gtc gct ctt ggg     192
Gly Arg Tyr Asn Leu Gln Pro Pro Ala Gly Gln Gly Val Ala Leu Gly
    50                  55                  60

tgg gcg gta tcc tgg gag aac agt aaa att cat tcc gct acg aca tgg     240
Trp Ala Val Ser Trp Glu Asn Ser Lys Ile His Ser Ala Thr Thr Trp
65                  70                  75                  80

agc gga cag ttc ttc tct gag tcg tct cca gtg att ctt act cag tgg     288
Ser Gly Gln Phe Phe Ser Glu Ser Ser Pro Val Ile Leu Thr Gln Trp
                85                  90                  95

ttg ttg tca tcg agc act gcg cgt ggg gac gta tgg gaa tcc aca ctt     336
Leu Leu Ser Ser Ser Thr Ala Arg Gly Asp Val Trp Glu Ser Thr Leu
            100                 105                 110

gtg ggg aat gat tcg ttt aca aag acg gcg ccg act gag cag cag atc     384
Val Gly Asn Asp Ser Phe Thr Lys Thr Ala Pro Thr Glu Gln Gln Ile
        115                 120                 125

gct cat gct caa ctc cat tgt cgc gca ccg agg ttg aag taa             426
Ala His Ala Gln Leu His Cys Arg Ala Pro Arg Leu Lys
    130                 135                 140

<210>  2
<211>  141
<212>  PRT
<213>  Pleurotus cornucopiae

<400>  2

Met Ser Asp Val Gln Ser Ser Leu Thr Gly Thr Trp Tyr Asn Glu Leu
1               5                   10                  15

Asn Ser Lys Met Glu Leu Thr Ala Asn Lys Asp Gly Thr Leu Thr Gly
            20                  25                  30

Lys Tyr Leu Ser Lys Val Gly Asp Val Tyr Val Pro Tyr Pro Leu Ser
        35                  40                  45

Gly Arg Tyr Asn Leu Gln Pro Pro Ala Gly Gln Gly Val Ala Leu Gly
    50                  55                  60

Trp Ala Val Ser Trp Glu Asn Ser Lys Ile His Ser Ala Thr Thr Trp
65                  70                  75                  80

Ser Gly Gln Phe Phe Ser Glu Ser Ser Pro Val Ile Leu Thr Gln Trp
                85                  90                  95

Leu Leu Ser Ser Ser Thr Ala Arg Gly Asp Val Trp Glu Ser Thr Leu
            100                 105                 110

Val Gly Asn Asp Ser Phe Thr Lys Thr Ala Pro Thr Glu Gln Gln Ile
        115                 120                 125

Ala His Ala Gln Leu His Cys Arg Ala Pro Arg Leu Lys
    130                 135                 140

<210>  3
<211>  432
<212>  DNA
<213>  Pleurotus cornucopiae

<220>
<221>  CDS
<222>  (1)..(432)

<400>  3
atg aaa gac gtc caa tct ctc ctc acc gga acc tgg tac aat gaa ctc       48
Met Lys Asp Val Gln Ser Leu Leu Thr Gly Thr Trp Tyr Asn Glu Leu
1               5                   10                  15

ggc tca aca atg aat ttg act gca aat aaa gac ggt tcg ctc acc gga       96
Gly Ser Thr Met Asn Leu Thr Ala Asn Lys Asp Gly Ser Leu Thr Gly
            20                  25                  30

acg tac cac tcc aac gtc ggc gag gtt ccc cca act tat cac ctt tct 144
Thr Tyr His Ser Asn Val Gly Glu Val Pro Pro Thr Tyr His Leu Ser
        35                  40                  45

ggc cgg tac aac ctc cag ccc ccc tcg ggt caa ggc gtt act ctg gga 192
Gly Arg Tyr Asn Leu Gln Pro Pro Ser Gly Gln Gly Val Thr Leu Gly
    50                  55                  60

tgg gcg gtg tct ttc gaa aac act agt gcg aat gtt cat tct gtc tca 240
Trp Ala Val Ser Phe Glu Asn Thr Ser Ala Asn Val His Ser Val Ser
65                  70                  75                  80

aca tgg agc ggg cag tac ttc tct gaa ccc gcc gag gtg atc ctc acc 288
Thr Trp Ser Gly Gln Tyr Phe Ser Glu Pro Ala Glu Val Ile Leu Thr
                85                  90                  95

cag tgg ctg ttg tca agg agc tct gag cgc gaa gat ttg tgg cag tcc 336
Gln Trp Leu Leu Ser Arg Ser Ser Glu Arg Glu Asp Leu Trp Gln Ser
            100                 105                 110

acc cat gtg ggg cat gat gag ttc agc aag aca aag cca acc aag gag 384
Thr His Val Gly His Asp Glu Phe Ser Lys Thr Lys Pro Thr Lys Glu
        115                 120                 125

aag att gcc cag gct caa ctc ctt cgt cgc ggg ttg aag ttc gag tga 432
Lys Ile Ala Gln Ala Gln Leu Leu Arg Arg Gly Leu Lys Phe Glu
    130                 135                 140

<210> 4
<211> 143
<212> PRT
<213> Pleurotus cornucopiae

<400> 4

Met Lys Asp Val Gln Ser Leu Leu Thr Gly Thr Trp Tyr Asn Glu Leu
1               5                   10                  15

Gly Ser Thr Met Asn Leu Thr Ala Asn Lys Asp Gly Ser Leu Thr Gly
            20                  25                  30

Thr Tyr His Ser Asn Val Gly Glu Val Pro Pro Thr Tyr His Leu Ser
        35                  40                  45

Gly Arg Tyr Asn Leu Gln Pro Pro Ser Gly Gln Gly Val Thr Leu Gly
    50                  55                  60

Trp Ala Val Ser Phe Glu Asn Thr Ser Ala Asn Val His Ser Val Ser
65                  70                  75                  80

Thr Trp Ser Gly Gln Tyr Phe Ser Glu Pro Ala Glu Val Ile Leu Thr
                85                  90                  95

Gln Trp Leu Leu Ser Arg Ser Ser Glu Arg Glu Asp Leu Trp Gln Ser
            100                 105                 110

Thr His Val Gly His Asp Glu Phe Ser Lys Thr Lys Pro Thr Lys Glu
        115                 120                 125

Lys Ile Ala Gln Ala Gln Leu Leu Arg Arg Gly Leu Lys Phe Glu
    130                 135                 140

<210>  5
<211>  552
<212>  DNA
<213>  Streptomyces avidinii

<220>
<221>  CDS
<222>  (1)..(552)

<400>  5
atg cgc aag atc gtc gtt gca gcc atc gcc gtt tcc ctg acc acg gtc      48
Met Arg Lys Ile Val Val Ala Ala Ile Ala Val Ser Leu Thr Thr Val
1               5                   10                  15

tcg att acg gcc agc gct tcg gca gac ccc tcc aag gac tcg aag gcc      96
Ser Ile Thr Ala Ser Ala Ser Ala Asp Pro Ser Lys Asp Ser Lys Ala
            20                  25                  30

cag gtc tcg gcc gcc gag gcc ggc atc acc ggc acc tgg tac aac cag      144
Gln Val Ser Ala Ala Glu Ala Gly Ile Thr Gly Thr Trp Tyr Asn Gln
        35                  40                  45

ctc ggc tcg acc ttc atc gtg acc gcg ggc gcc gac ggc gcc ctg acc      192
Leu Gly Ser Thr Phe Ile Val Thr Ala Gly Ala Asp Gly Ala Leu Thr
    50                  55                  60

gga acc tac gag tcg gcc gtc ggc aac gcc gag agc cgc tac gtc ctg      240
Gly Thr Tyr Glu Ser Ala Val Gly Asn Ala Glu Ser Arg Tyr Val Leu
65                  70                  75                  80

acc ggt cgt tac gac agc gcc ccg gcc acc gac ggc agc ggc acc gcc      288
Thr Gly Arg Tyr Asp Ser Ala Pro Ala Thr Asp Gly Ser Gly Thr Ala
                85                  90                  95

ctc ggt tgg acg gtg gcc tgg aag aat aac tac cgc aac gcc cac tcc      336
Leu Gly Trp Thr Val Ala Trp Lys Asn Asn Tyr Arg Asn Ala His Ser
            100                 105                 110

gcg acc acg tgg agc ggc cag tac gtc ggc ggc gcc gag gcg agg atc      384
Ala Thr Thr Trp Ser Gly Gln Tyr Val Gly Gly Ala Glu Ala Arg Ile
        115                 120                 125

aac acc cag tgg ctg ctg acc tcc ggc acc acc gag gcc aac gcc tgg      432
Asn Thr Gln Trp Leu Leu Thr Ser Gly Thr Thr Glu Ala Asn Ala Trp
    130                 135                 140

aag tcc acg ctg gtc ggc cac gac acc ttc acc aag gtg aag ccg tcc      480
Lys Ser Thr Leu Val Gly His Asp Thr Phe Thr Lys Val Lys Pro Ser
145                 150                 155                 160

gcc gcc tcc atc gac gcg gcg aag aag gcc ggc gtc aac aac ggc aac      528
Ala Ala Ser Ile Asp Ala Ala Lys Lys Ala Gly Val Asn Asn Gly Asn
                165                 170                 175

ccg ctc gac gcc gtt cag cag tag                                    552
Pro Leu Asp Ala Val Gln Gln
            180

<210>  6
<211>  183
<212>  PRT
<213>  Streptomyces avidinii

<400>  6

Met Arg Lys Ile Val Val Ala Ala Ile Ala Val Ser Leu Thr Thr Val
1               5                   10                  15

Ser Ile Thr Ala Ser Ala Ser Ala Asp Pro Ser Lys Asp Ser Lys Ala
            20                  25                  30

Gln Val Ser Ala Ala Glu Ala Gly Ile Thr Gly Thr Trp Tyr Asn Gln
        35                  40                  45

Leu Gly Ser Thr Phe Ile Val Thr Ala Gly Ala Asp Gly Ala Leu Thr
    50                  55                  60

Gly Thr Tyr Glu Ser Ala Val Gly Asn Ala Glu Ser Arg Tyr Val Leu
65                  70                  75                  80

Thr Gly Arg Tyr Asp Ser Ala Pro Ala Thr Asp Gly Ser Gly Thr Ala
                85                  90                  95

Leu Gly Trp Thr Val Ala Trp Lys Asn Asn Tyr Arg Asn Ala His Ser
            100                 105                 110

Ala Thr Thr Trp Ser Gly Gln Tyr Val Gly Gly Ala Glu Ala Arg Ile
        115                 120                 125

Asn Thr Gln Trp Leu Leu Thr Ser Gly Thr Thr Glu Ala Asn Ala Trp
    130                 135                 140

Lys Ser Thr Leu Val Gly His Asp Thr Phe Thr Lys Val Lys Pro Ser
145                 150                 155                 160

Ala Ala Ser Ile Asp Ala Ala Lys Lys Ala Gly Val Asn Asn Gly Asn
                165                 170                 175

Pro Leu Asp Ala Val Gln Gln
            180

<210>  7
<211>  459

<212>  DNA
<213>  Gallus gallus domesticus

<220>
<221>  CDS
<222>  (1)..(459)

<400>  7
atg gtg cac gca acc tcc ccg ctg ctg ctg ctg ctg ctg ctc agc ctg      48
Met Val His Ala Thr Ser Pro Leu Leu Leu Leu Leu Leu Leu Ser Leu
1               5                   10                  15

gct ctg gtg gct ccc ggc ctc tct gcc aga aag tgc tcg ctg act ggg      96
Ala Leu Val Ala Pro Gly Leu Ser Ala Arg Lys Cys Ser Leu Thr Gly
            20                  25                  30

aaa tgg acc aac gat ctg ggc tcc aac atg acc atc ggg gct gtg aac      144
Lys Trp Thr Asn Asp Leu Gly Ser Asn Met Thr Ile Gly Ala Val Asn
        35                  40                  45

agc aga ggt gaa ttc aca ggc acc tac atc aca gcc gta aca gcc aca      192
Ser Arg Gly Glu Phe Thr Gly Thr Tyr Ile Thr Ala Val Thr Ala Thr
    50                  55                  60

tca aat gag atc aaa gag tca cca ctg cat ggg aca caa aac acc atc      240
Ser Asn Glu Ile Lys Glu Ser Pro Leu His Gly Thr Gln Asn Thr Ile
65                  70                  75                  80

aac aag agg acc cag ccc acc ttt ggc ttc acc gtc aat tgg aag ttt      288
Asn Lys Arg Thr Gln Pro Thr Phe Gly Phe Thr Val Asn Trp Lys Phe
                85                  90                  95

tca gag tcc acc act gtc ttc acg ggc cag tgc ttc ata gac agg aat      336
Ser Glu Ser Thr Thr Val Phe Thr Gly Gln Cys Phe Ile Asp Arg Asn
            100                 105                 110

ggg aag gag gtc ctg aag acc atg tgg ctg ctg cgg tca agt gtt aat      384
Gly Lys Glu Val Leu Lys Thr Met Trp Leu Leu Arg Ser Ser Val Asn
        115                 120                 125

gac att ggt gat gac tgg aaa gct acc agg gtc ggc atc aac atc ttc      432
Asp Ile Gly Asp Asp Trp Lys Ala Thr Arg Val Gly Ile Asn Ile Phe
    130                 135                 140

act cgc ctg cgc aca cag aag gag tga                                  459
Thr Arg Leu Arg Thr Gln Lys Glu
145                 150

<210>  8
<211>  152
<212>  PRT
<213>  Gallus gallus domesticus

<400>  8

Met Val His Ala Thr Ser Pro Leu Leu Leu Leu Leu Leu Leu Ser Leu
1               5                   10                  15

Ala Leu Val Ala Pro Gly Leu Ser Ala Arg Lys Cys Ser Leu Thr Gly
            20                  25                  30

Lys Trp Thr Asn Asp Leu Gly Ser Asn Met Thr Ile Gly Ala Val Asn
        35                  40                  45

Ser Arg Gly Glu Phe Thr Gly Thr Tyr Ile Thr Ala Val Thr Ala Thr
    50                  55                  60

Ser Asn Glu Ile Lys Glu Ser Pro Leu His Gly Thr Gln Asn Thr Ile
65                  70                  75                  80

Asn Lys Arg Thr Gln Pro Thr Phe Gly Phe Thr Val Asn Trp Lys Phe
                85                  90                  95

Ser Glu Ser Thr Thr Val Phe Thr Gly Gln Cys Phe Ile Asp Arg Asn
            100                 105                 110

Gly Lys Glu Val Leu Lys Thr Met Trp Leu Leu Arg Ser Ser Val Asn
        115                 120                 125

Asp Ile Gly Asp Asp Trp Lys Ala Thr Arg Val Gly Ile Asn Ile Phe
    130                 135                 140

Thr Arg Leu Arg Thr Gln Lys Glu
145                 150

<210>  9
<211>  426
<212>  DNA
<213>  Artificial

<220>
<223>  TM2 N115C

<400>  9
atg tca gac gtt caa tct tca ctc acc gga acc tgg tac aat gaa ctc        48
Met Ser Asp Val Gln Ser Ser Leu Thr Gly Thr Trp Tyr Asn Glu Leu
1               5                   10                  15

aac tcc aag atg gaa ttg act gca aac aaa gac ggt act ctc act gga        96
Asn Ser Lys Met Glu Leu Thr Ala Asn Lys Asp Gly Thr Leu Thr Gly
            20                  25                  30

aag tac ctc tcc aaa gtt ggg gat gtc tac gtg ccc tac cca ctc tct       144
Lys Tyr Leu Ser Lys Val Gly Asp Val Tyr Val Pro Tyr Pro Leu Ser
        35                  40                  45

ggt cgc tat aac ctc caa ccc ccc gcg gga caa ggc gtc gct ctt ggg       192
Gly Arg Tyr Asn Leu Gln Pro Pro Ala Gly Gln Gly Val Ala Leu Gly
    50                  55                  60

tgg gcg gta tcc tgg gag aac agt aaa att cat tcc gct acg aca tgg       240
Trp Ala Val Ser Trp Glu Asn Ser Lys Ile His Ser Ala Thr Thr Trp
65                  70                  75                  80

agc gga cag ttc ttc tct gag tcg tct cca gtg att ctt act cag tgg     288
Ser Gly Gln Phe Phe Ser Glu Ser Ser Pro Val Ile Leu Thr Gln Trp
                85                  90                  95

ttg ttg tca tcg agc act gcg cgt ggg gac gta tgg gaa tcc aca ctt     336
Leu Leu Ser Ser Ser Thr Ala Arg Gly Asp Val Trp Glu Ser Thr Leu
            100                 105                 110

gtg ggg tgt gat tcg ttt aca aag acg gcg ccg act gag cag cag atc     384
Val Gly Cys Asp Ser Phe Thr Lys Thr Ala Pro Thr Glu Gln Gln Ile
        115                 120                 125

gct cat gct caa ctc cat tgt cgc gca ccg agg ttg aag taa             426
Ala His Ala Gln Leu His Cys Arg Ala Pro Arg Leu Lys
    130                 135                 140


<210>  10
<211>  141
<212>  PRT
<213>  Artificial

<220>
<223>  Synthetic Construct

<400>  10

Met Ser Asp Val Gln Ser Ser Leu Thr Gly Thr Trp Tyr Asn Glu Leu
1               5                   10                  15

Asn Ser Lys Met Glu Leu Thr Ala Asn Lys Asp Gly Thr Leu Thr Gly
            20                  25                  30

Lys Tyr Leu Ser Lys Val Gly Asp Val Tyr Val Pro Tyr Pro Leu Ser
        35                  40                  45

Gly Arg Tyr Asn Leu Gln Pro Pro Ala Gly Gln Gly Val Ala Leu Gly
    50                  55                  60

Trp Ala Val Ser Trp Glu Asn Ser Lys Ile His Ser Ala Thr Thr Trp
65                  70                  75                  80

Ser Gly Gln Phe Phe Ser Glu Ser Ser Pro Val Ile Leu Thr Gln Trp
                85                  90                  95

Leu Leu Ser Ser Ser Thr Ala Arg Gly Asp Val Trp Glu Ser Thr Leu
            100                 105                 110

Val Gly Cys Asp Ser Phe Thr Lys Thr Ala Pro Thr Glu Gln Gln Ile
        115                 120                 125

Ala His Ala Gln Leu His Cys Arg Ala Pro Arg Leu Lys
    130                 135                 140

<210>  11
<211>  25
<212>  DNA
<213>  Artificial

<220>
<223>  Primer Tm2NtermPci

<400>  11
aaaacatgtc agacgttcaa tcttc                                              25


<210>  12
<211>  28
<212>  DNA
<213>  Artificial

<220>
<223>  Primer Tm2CtermBam

<400>  12
tttggatcct tacttcaacc tcggtgcg                                           28


<210>  13
<211>  21
<212>  DNA
<213>  Artificial

<220>
<223>  forward primer for PCR TM2 N115C

<400>  13
cttgtggggt gtgattcgtt t                                                  21


<210>  14
<211>  21
<212>  DNA
<213>  Artificial

<220>
<223>  reverse primer for PCR TM2 N115C

<400>  14
aaacgaatca caccccacaa g                                                  21


<210>  15
<211>  5
<212>  PRT
<213>  Artificial

<220>
<223>  linker for fusion protein

<400>  15

Gly Gly Gly Gly Ser
1               5


<210>  16

<211>  5
<212>  PRT
<213>  Artificial

<220>
<223>  linker for fusion protein

<400>  16

Gly Gly Ser Gly Gly
1               5


<210>  17
<211>  5
<212>  PRT
<213>  Artificial

<220>
<223>  linker for fusion protein

<400>  17

Gly Ala Ser Ala Gly
1               5


<210>  18
<211>  5
<212>  PRT
<213>  Artificial

<220>
<223>  linker for fusion protein

<400>  18

Gly Ser Gly Ala Ala
1               5


<210>  19
<211>  5
<212>  PRT
<213>  Artificial

<220>
<223>  linker for fusion protein

<400>  19

Gly Ser Gly Ser Ala
1               5


<210>  20
<211>  6
<212>  PRT
<213>  Artificial

<220>
<223>  linker for fusion protein

<400>  20

Gly Gly Gly Gly Ser Gly
1               5


<210>  21
<211>  7
<212>  PRT
<213>  Artificial

<220>
<223>  linker for fusion protein

<400>  21

Gly Gly Gly Ser Gly Gly Ser
1               5


<210>  22
<211>  8
<212>  PRT
<213>  Artificial

<220>
<223>  linker for fusion protein

<400>  22

Gly Gly Ser Gly Gly Gly Gly Ser
1               5


<210>  23
<211>  9
<212>  PRT
<213>  Artificial

<220>
<223>  linker for fusion protein

<400>  23

Ala Ala Ala Ala Gly Ser Gly Ala Ala
1               5


<210>  24
<211>  15
<212>  PRT
<213>  Artificial

<220>
<223>  linker for fusion protein

<400>  24

Gly Gly Gly Gly Ser Gly Gly Gly Gly Ser Gly Gly Gly Gly Ser
1               5                   10                  15


<210>  25

<211>  25
<212>  PRT
<213>  Artificial

<220>
<223>  linker for fusion protein

<400>  25

Gly Gly Gly Gly Ser Gly Gly Gly Gly Ser Gly Gly Gly Gly Ser Gly
1               5                   10                  15

Gly Gly Gly Ser Gly Gly Gly Gly Ser
            20                  25